(19) Europäisches Patentamt
European Patent Office
Office européen des brevets

(11) EP 1 917 233 B1

(12) **EUROPEAN PATENT SPECIFICATION**

(45) Date of publication and mention of the grant of the patent:
**17.12.2008 Bulletin 2008/51**

(21) Application number: **06802161.7**

(22) Date of filing: **23.08.2006**

(51) Int Cl.:
***C07C 215/68*** *(2006.01)* ***C07D 213/06*** *(2006.01)*
***A61K 31/137*** *(2006.01)* ***A61K 31/44*** *(2006.01)*

(86) International application number:
**PCT/US2006/032926**

(87) International publication number:
**WO 2007/024954 (01.03.2007 Gazette 2007/09)**

(54) **ANILINOHEXAFLUOROISOPROPANOL COMPOUNDS**
ANILINOHEXAFLUOROISOPROPANOL VERBINDUNGEN
ANILINOHEXAFLUOROISOPROPANOLS

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **25.08.2005 US 711267 P**

(43) Date of publication of application:
**07.05.2008 Bulletin 2008/19**

(73) Proprietor: **SmithKline Beecham Corporation**
**Philadelphia, PA 19101 (US)**

(72) Inventors:
- **COLLINS, Jon Loren**
  **Research Triangle Park, NC 27709 (US)**
- **STEWART, Eugene Lee**
  **Research Triangle Park, NC 27709 (US)**
- **ZUERCHER, William**
  **Research Triangle Park, 27709 (US)**
- **CHAO, Esther**
  **Research Triangle Park, NC 27709 (US)**
- **WIETHE, Robert**
  **Research Triangle Park, NC 27709 (US)**
- **CARAVELLA, Justin**
  **Research Triangle Park, NC 27709 (US)**

(74) Representative: **Sewell, Richard Charles et al**
**GlaxoSmithKline**
**Corporate Intellectual Property**
**CN925.1**
**980 Great West Road**
**Brentford, Middlesex TW8 9GS (GB)**

(56) References cited:
**WO-A-03/099769** **WO-A2-00/54759**

- **MASCIARDI, RAFFAELLO ET AL: "Regioselective Friedel-Crafts alkylation of anilines and amino-substituted heteroarenes with hexafluoroacetone sesquihydrate" EUROPEAN JOURNAL OF ORGANIC CHEMISTRY, vol. 21, 2003, pages 4286-4291, XP002414515**

EP 1 917 233 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

This invention relates to compounds that are modulators of the liver X receptors (LXRs), and also to the methods for the making and use of such compounds.

### BACKGROUND OF THE INVENTION

The liver X receptors (LXRs), LXRα and LXRβ, are ligand-activated transcription factors of the nuclear hormone receptor superfamily (Peet, DJ et al., Curr. Opin. Genet. Dev., 1998, 8(5)571-575.) The identification of cholesterol metabolites as the natural ligands for the LXRs suggested that the biological role of the receptors is to regulate cholesterol homeostasis. Studies in mice lacking LXRα, but not LXRβ, demonstrated that LXRα (-/-) mice were unable to effectively convert cholesterol to bile acid. The LXRα (-/-) mice also exhibited accumulation of cholesterol in the liver, as well as elevated levels of serum low-density lipoporoteins, when compared to wild type animals. These and other data strongly support the hypothesis that LXRs play a significant role in regulating cholesterol metabolism in mammals. See, for example, Peet, DJ et al., Cell, 1998, 93(5):693-704.

Identification of potent and selective dual LXRα/β agonists has provided non-steroidal chemical tools to further decipher the biology of the LXRs. See, for example Schultz, JR et al., Gene Dev., 2000, 14(22):2831-2838 and Collins, JL et al., J. Med. Chem., 2002, 45(10):1963-1966. Cumulative data from extensive in vivo and in vitro studies suggest that LXR agonists are candidates for the treatment of cardiovascular disease. See, for example, Tontonoz, P. et al., Mol. Endocrinol., 2003, 17(6):985-993. More recent developments suggest that ligands for LXRs may also provide therapeutic opportunities for the treatment of inflammation, diabetes, and neurodegenerative diseases. See, for example, Joseph, SB et al., Nat. Med., 2003, 9(2):213-219; Stulnig, TM et al., Mol. Pharmacol., 2002, 62(6):1299-1305; and Wang, L et al., Proc. Natl. Acad. Sci. USA, 2002, 99(21):13878-13883. However, since LXRs regulate the expression of many genes involved in cholesterol homeostasis, as well as macrophage innate immune responses, glucose metabolism, and fatty acid metabolism, and LXRs are expressed in many different tissues, it is believed that LXR modulators may provide the best therapeutic opportunites for most diseases or disorders.

### SUMMARY OF INVENTION

Briefly, in one aspect, the present invention provides compounds of formula (I)

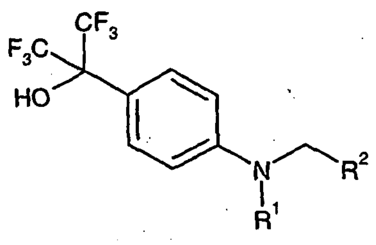

(I)

or a salt or solvate thereof, wherein
$R^1$ is $C_1$-$C_4$ alkyl ; and
$R^2$ is phenyl, or pyridinyl, wherein said phenyl is optionally substituted with one or more substituents, each independently selected from hydroxyl, alkoxy, halogen, haloalkoxy, and $-O(CH_2)_2OH$.

Another aspect of the present invention provides a pharmaceutical composition comprising a compound of the present invention.

Another aspect of the present invention provides a compound of the present invention for use as an active therapeutic substance.

Another aspect of the present invention provides a compound of the present invention for use in the treatment of conditions or disorders that respond to LXR modulator activity.

Another aspect of the present invention provides a compound of the present invention for use in the treatment of type 2 diabetes mellitus, cardiovascular disease, dyslipidemia, artherosclerosis, peripheral vascular disease, inflammation, cancer, and diseases of the central nervous system.

Another aspect of the present invention provides the use of a compound of the present invention in the manufacture of a medicament for use in the treatment of conditions or disorders that respond to LXR modulator activity.

Another aspect of the present invention provides the use of a compound of the present invention in the manufacture of a medicament for use in the treatment of type 2 diabetes mellitus, cardiovascular disease, dyslipidemia, artherosclerosis, peripheral vascular disease, inflammation, cancer, and diseases of the central nervous system.

## DETAILED DESCRIPTION OF THE PREFERRED EMBODIMENT

Terms are used within their accepted meanings. The following definitions are meant to clarify, but not limit, the terms defined.

In one embodiment, the present invention provides compounds of formula I, or a salt or solvate thereof, wherein $R^1$ is methyl or butyl, and $R^2$ is phenyl, or pyridinyl, wherein said phenyl is optionally substituted with one to three substituents, each independently selected from hydroxyl, methoxy, Cl, F, $-OCF_3$, and $-O(CH_2)_2OH$.

Another embodiment of the present invention provides a compound of formula I or a salt or solvate thereof, wherein $R^1$ is butyl, and $R^2$ is phenyl substituted with three substituents, each independently selected from hydroxyl, methoxy, and Cl.

In another embodiment, the present invention provides a compound selected from the group consisting of:

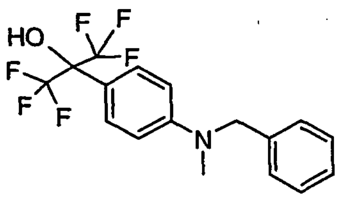
;
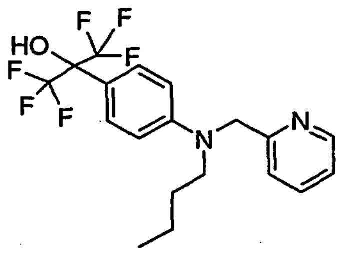
;
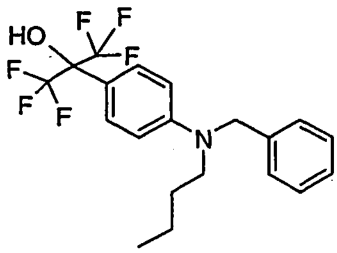
;
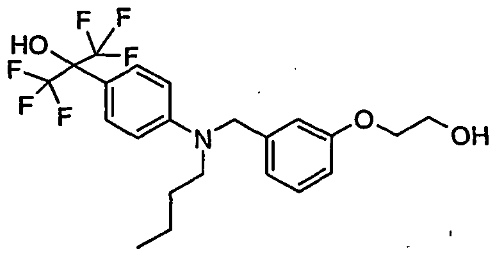
;

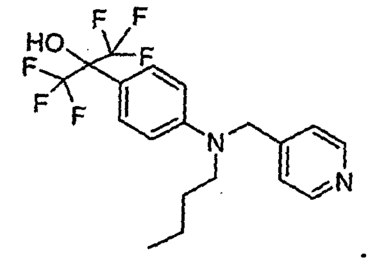

;

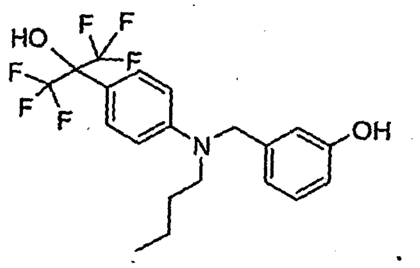

;

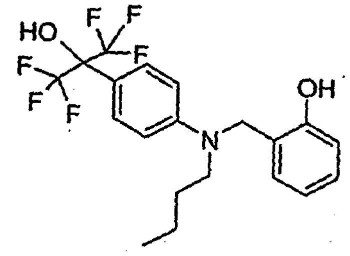

;

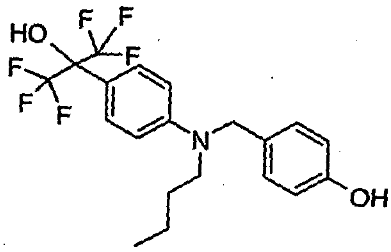

;

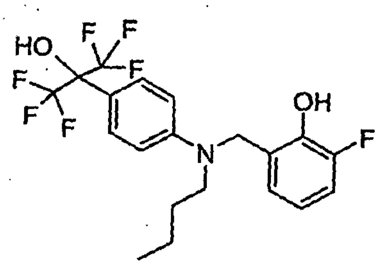

;

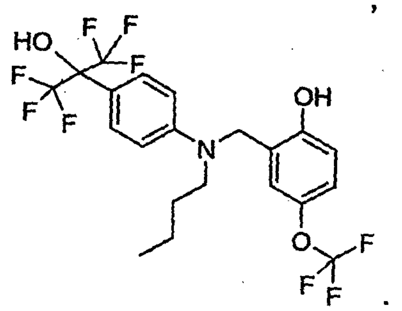

;

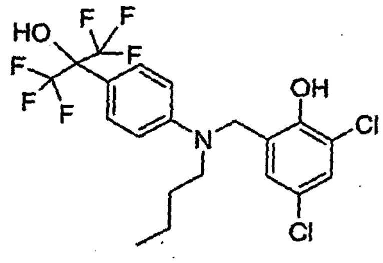

;

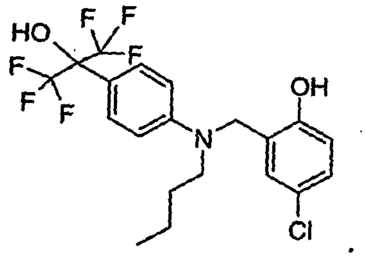

;

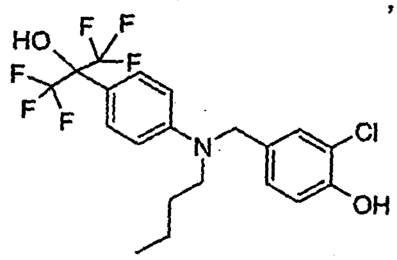

;

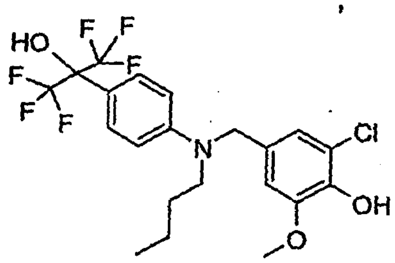

;

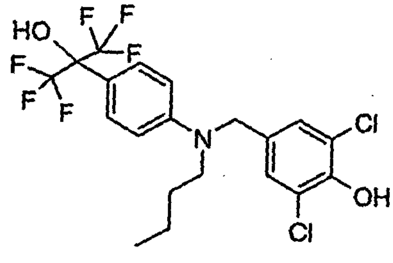

;

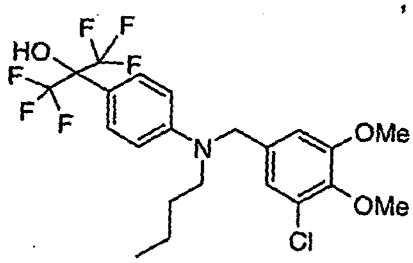

;

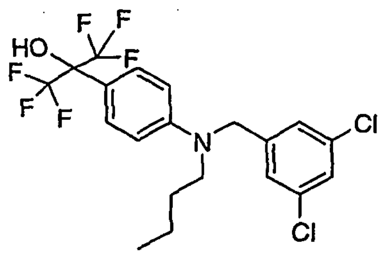

; and

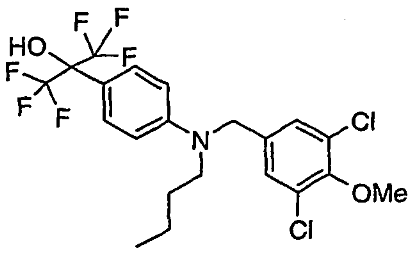

;

or a salt or solvate thereof.

In another embodiment, the present invention provides a compound of formula (I-A):

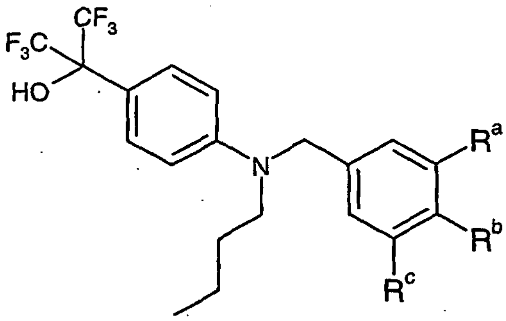

(I-A)

or a salt or solvate thereof, wherein

$R^a$ is Cl or methoxy;

$R^b$ is hydroxyl or methoxy; and

$R^c$ is Cl or methoxy.

Another embodiment of the present invention provides a compound of formula I-A, wherein $R^a$ is Cl, $R^b$ is hydroxyl, and $R^c$ is methoxy.

Another embodiment of the present invention provides a compound of formula I-A, wherein $R^a$ is Cl, $R^b$ is hydroxyl, and $R^c$ is Cl.

Another embodiment of the present invention provides a compound of formula I-A, wherein $R^a$ is methoxy, $R^b$ is methoxy, and $R^c$ is Cl.

A further embodiment of the present invention provides a compound selected from the group consisting of:

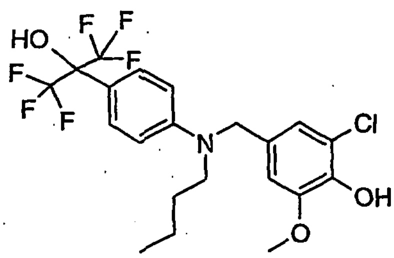

;

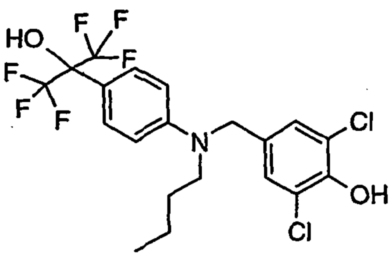

;

and

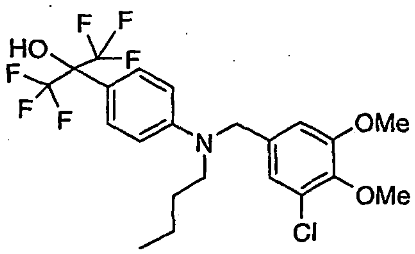

;

or a salt or solvate thereof.

While the embodiments or preferred groups for each variable have generally been listed above separately for each variable, compounds of this invention include those in which several of each variable in formula (I) are selected from the embodiments or preferred groups for each variable. Therefore, this invention is intended to include all combinations of embodiments and preferred groups.

The compounds of the present invention modulate the function of one or more nuclear receptor(s). Particularly, the compounds of the present invention modulate the liver X receptors ("LXRs"). The present invention includes compounds that are partial agonists, selective agonists, antagonists, or partial antagonists of LXRα and LXRβ. Compounds of the present invention are useful in the treatment of LXR-associated diseases and conditions. For example, a disease or condition that is prevented, alleviated, or cured through the modulation of the function or activity of LXRs. Such modulation may be isolated within certain tissues or widespread throughout the body of the subject being treated.

As used herein, the term "treatment" refers to alleviating the specified condition, eliminating or reducing the symptoms of the condition, slowing or eliminating the progression of the condition and preventing or delaying the initial occurrence of the condition in a subject, or reoccurrance of the condition in a previously afflicted subject.

One embodiment of the present invention is the use of the compounds of the present invention for the preparation of a medicament for the treatment of a variety of disorders including, but not limited to, cardiovascular disease, atherosclerosis, dyslipidemia, peripheral vascular disease, type 2 diabetes, inflammation, Castleman's disease, asthma, rheumatoid arthritis, juvenile idiopathic arthritis, inflammatory bowel disease, ulcerative colitis, cholestasis, psorasis, systemic lupus erythematosus, cancers such as myeloma and cachexia, and diseases of the central nervous system such as Alzheimer's disease and bipolar disorder.

The compounds of the present invention may crystallize in more than one form, a characteristic known as polymorphism, and such polymorphic forms ("polymorphs") are within the scope of the present invention. Polymorphism generally may occur as a response to changes in temperature, pressure, or both. Polymorphism may also result from variations in the crystallization process. Polymorphs may be distinguished by various physical characteristics known in the art such as x-ray diffraction patterns, solubility, and melting point.

Certain of the compounds described herein contain one or more chiral centers, or may otherwise be capable of existing as multiple stereoisomers. The scope of the present invention includes mixtures of stereoisomers as well as purified enantiomers or enantiomerically/diastereomerically enriched mixtures. Also included within the scope of the invention are the individual isomers of the compounds represented by formula (I), as well as any wholly or partially equilibrated mixtures thereof. The present invention also includes the individual isomers of the compounds represented by the formulas above as mixtures with isomers thereof in which one or more chiral centers are inverted.

Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in a compound of the present invention. Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

As used herein, the term "solvate" refers to a complex of variable stoichiometry formed by a solute (in this invention, a compound of the present invention) and a solvent. Such solvents, for the purpose of the invention, should not interfere with the biological activity of the solute. Non-limiting examples of suitable solvents include, but are not limited to water, methanol, ethanol, and acetic acid. Preferably the solvent used is a pharmaceutically acceptable solvent.

Non-limiting examples of suitable pharmaceutically acceptable solvents include water, ethanol, and acetic acid. Most preferably the solvent used is water.

As used herein, the term "physiologically functional derivative" refers to any pharmaceutically acceptable derivative of a compound of the present invention that, upon administration to a mammal, is capable of providing (directly or indirectly) a compound of the present invention or an active metabolite thereof. Such derivatives, for example, esters and amides, will be clear to those skilled in the art, without undue experimentation. Reference may be made to the teaching of Burger's Medicinal Chemistry And Drug Discovery, 5th Edition, Vol 1: Principles and Practice to the extent that it teaches physiologically functional derivatives.

As used herein, the term "effective amount" means that amount of a drug or pharmaceutical agent that will elicit the biological or medical response of a tissue, system, animal, or human that is being sought, for instance, by a researcher or clinician. The biological or medical response may be considered a prophylactic response or a treatment response. The term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. For use in therapy, therapeutically effective amounts of a compound of the present invention may be administered as the raw chemical. Additionally, the active ingredient may be presented as a pharmaceutical composition.

Accordingly, the invention further provides pharmaceutical compositions that include effective amounts of compounds of the present invention and one or more pharmaceutically acceptable carriers, diluents, or excipients. The compounds of the present invention are as herein described. The carrier(s), diluent(s) or excipient(s) must be acceptable, in the sense of being compatible with the other ingredients of the formulation and not deleterious to the recipient of the pharmaceutical composition.

In accordance with another aspect of the invention there is also provided a process for the preparation of a pharmaceutical formulation including admixing a compound of the present invention with one or more pharmaceutically acceptable carriers, diluents or excipients.

A therapeutically effective amount of a compound of the present invention will depend upon a number of factors. For example, the species, age, and weight of the recipient, the precise condition requiring treatment and its severity, the nature of the formulation, and the route of administration are all factors to be considered. The therapeutically effective amount ultimately should be at the discretion of the attendant physician or veterinarian. Usually the effective amount should be in the range of 0.1 to 10 mg/kg body weight per day. Thus, for a 70 kg adult mammal the actual amount per day would usually be from 7 to 700 mg. This amount may be given in a single dose per day or in a number (such as two, three, four, five, or more) of sub-doses per day such that the total daily dose is the same. An effective amount of a salt or solvate thereof, may be determined as a proportion of the effective amount of the compound of the present invention *per se.* Similar dosages should be appropriate for treatment of the other conditions referred to herein.

Pharmaceutical formulations may be presented in unit dose forms containing a predetermined amount of active ingredient per unit dose. Such a unit may contain, as a non-limiting example, 0.5 mg to 1 g of a compound of the present invention, depending on the condition being treated, the route of administration, and the age, weight, and condition of the patient. Preferred unit dosage formulations are those containing a daily dose or sub-dose, as herein above recited, or an appropriate fraction thereof, of an active ingredient. Such pharmaceutical formulations may be prepared by any of the methods well known in the pharmacy art.

Pharmaceutical formulations may be adapted for administration by any appropriate route, for example by an oral (including buccal or sublingual), rectal, nasal, topical (including buccal, sublingual or transdermal), vaginal, or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions, each with aqueous or non-aqueous liquids; edible foams or whips; or oil-in-water liquid emulsions or water-in-oil liquid emulsions. For instance, for oral administration in the form of a tablet or capsule, the active drug component may be combined with an oral, non-toxic pharmaceutically acceptable inert carrier such as ethanol, glycerol, water, and the like. Generally, powders are prepared by comminuting the compound to a suitable fine size and mixing with an appropriate pharmaceutical carrier such as an edible carbohydrate, as, for example, starch or mannitol. Flavorings, preservatives, dispersing agents, and coloring agents may also be present.

Capsules may be made by preparing a powder, liquid, or suspension mixture and encapsulating with gelatin or some other appropriate shell material. Glidants and lubricants such as colloidal silica, talc, magnesium stearate, calcium stearate, or solid polyethylene glycol may be added to the mixture before the encapsulation. A disintegrating or solubilizing agent such as agar-agar, calcium carbonate or sodium carbonate may also be added to improve the availability of the medicament when the capsule is ingested. Moreover, when desired or necessary, suitable binders, lubricants, disintegrating agents, and coloring agents may also be incorporated into the mixture. Examples of suitable binders

include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants useful in these dosage forms include, for example, sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

Tablets may be formulated, for example, by preparing a powder mixture, granulating or slugging, adding a lubricant and disintegrant, and pressing into tablets. A powder mixture may be prepared by mixing the compound, suitably comminuted, with a diluent or base as described above. Optional ingredients include binders such as carboxymethylcellulose, aliginates, gelatins, or polyvinyl pyrrolidone, solution retardants such as paraffin, resorption accelerators such as a quaternary salt, and/or absorption agents such as bentonite, kaolin, or dicalcium phosphate. The powder mixture may be wet-granulated with a binder such as syrup, starch paste, acadia mucilage or solutions of cellulosic or polymeric materials, and forcing through a screen. As an alternative to granulating, the powder mixture may be run through the tablet machine and the result is imperfectly formed slugs broken into granules. The granules may be lubricated to prevent sticking to the tablet forming dies by means of the addition of stearic acid, a stearate salt, talc or mineral oil. The lubricated mixture is then compressed into tablets. The compounds of the present invention may also be combined with a free flowing inert carrier and compressed into tablets directly without going through the granulating or slugging steps. A clear or opaque protective coating consisting of a sealing coat of shellac, a coating of sugar or polymeric material, and a polish coating of wax may be provided. Dyestuffs may be added to these coatings to distinguish different unit dosages.

Oral fluids such as solutions, syrups, and elixirs may be prepared in dosage unit form so that a given quantity contains a predetermined amount of the compound. Syrups may be prepared, for example, by dissolving the compound in a suitably flavored aqueous solution, while elixirs may be prepared through the use of a non-toxic alcoholic vehicle. Suspensions may be formulated generally by dispersing the compound in a non-toxic vehicle. Solubilizers and emulsifiers such as ethoxylated isostearyl alcohols and polyoxy ethylene sorbitol ethers, preservatives; flavor additives such as peppermint oil, or natural sweeteners, saccharin, or other artificial sweeteners; and the like may also be added.

Where appropriate, dosage unit formulations for oral administration may be microencapsulated. The formulation may also be prepared to prolong or sustain the release as for example by coating or embedding particulate material in polymers, wax or the like.

The compounds of the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

The compounds of the present invention may also be delivered by the use of monoclonal antibodies as individual carriers to which the compound molecules are coupled.

The compounds may also be coupled with soluble polymers as targetable drug carriers. Such polymers may include polyvinylpyrrolidone (PVP), pyran copolymer, polyhydroxypropylmethacrylamide-phenol, polyhydroxyethyl-aspartamidephenol, or polyethyleneoxidepolylysine substituted with palmitoyl residues. Furthermore, the compounds may be coupled to a class of biodegradable polymers useful in achieving controlled release of a drug; for example, polylactic acid, polyepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986), incorporated herein by reference as related to such delivery systems.

Pharmaceutical formulations adapted for topical administration may be formulated as ointments, creams, suspensions, lotions, powders, solutions, pastes, gels, sprays, aerosols, or oils.

For treatments of the eye or other external tissues, for example mouth and skin, the formulations may be applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles, and mouthwashes.

Pharmaceutical formulations adapted for nasal administration, where the carrier is a solid, include a coarse powder having a particle size for example in the range 20 to 500 microns. The powder is administered in the manner in which snuff is taken, i.e., by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists, which

may be generated by means of various types of metered dose pressurized aerosols, nebulizers, or insufflators.

Pharmaceutical formulations adapted for rectal administration may be presented as suppositories or as enemas.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and solutes that render the formulation isotonic with the blood of the intended recipient; and aqueous and non-aqueous sterile suspensions which may include suspending agents and thickening agents. The formulations may be presented in unit-dose or multi-dose containers, for example sealed ampules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

In addition to the ingredients particularly mentioned above, the formulations may include other agents conventional in the art having regard to the type of formulation in question. For example, formulations suitable for oral administration may include flavoring or coloring agents.

The compounds of the present invention and their salts or solvates thereof, may be employed alone or in combination with other therapeutic agents for the treatment of the above-mentioned conditions. The compound(s) of the present invention and the other pharmaceutically active agent(s) may be administered together or separately and, when administered separately, administration may occur simultaneously or sequentially, in any order. The amounts of the compound(s) of the present invention and the other pharmaceutically active agent(s) and the relative timings of administration will be selected in order to achieve the desired combined therapeutic effect. The administration in combination of a compound of the present invention with other treatment agents may be in combination by administration concomitantly in: (1) a unitary pharmaceutical composition including both compounds; or (2) separate pharmaceutical compositions each including one of the compounds. Alternatively, the combination may be administered separately in a sequential manner wherein one treatment agent is administered first and the other second or vice versa. Such sequential administration may be close in time or remote in time.

The compounds of the present invention may be used in the manufacture of a medicament for the treatment of a variety of disorders and conditions and, as such, the compounds of the present invention may be used in combination with a variety of other suitable therapeutic agents useful in the treatment of those disorders or conditions. Non-limiting examples include combinations of the present invention with anti-diabetic agents, anti-obesity agents, anti-inflammatory agents, anti-anxiety agents, anti-depressants, anti-hypertensive agents, anti-platelet agents, anti-thrombotic and thrombolytic agents, cardiac glycosides, cholesterol or lipid lowering agents, phosphodiesterase inhibitors, kinase inhibitors, thyroid mimetics, viral therapies, cognitive disorder therapies, sleeping disorder therapies, cytotoxic agents, radiation therapy, anti-proliferative agents, and anti-tumor agents. Additionally, the compounds of the present invention may be combined with nutritional supplements such as amino acids, triglycerides, vitamins, minerals, creatine, piloic acid, carnitine, or coenzyme Q10.

In particular, the compounds of the present invention are believed useful, either alone or in combination with other agents, in the manufacture of a medicament for the treatment of cardiovascular disease, atherosclerosis, dyslipidemia, peripheral vascular disease, type 2 diabetes, inflammation, Castleman's disease, asthma, rheumatoid arthritis, juvenile idiopathic arthritis, inflammatory bowel disease, ulcerative colitis, cholestasis, psorasis, systemic lupus erythematosus, cancers such as myeloma and cachexia, and diseases of the central nervous system such as Alzheimer's disease and bipolar disorder.

The compounds of this invention may be made by a variety of methods, including well-known standard synthetic methods. Illustrative general synthetic methods are set out below and then specific compounds of the invention are prepared in the working Examples.

In all of the schemes described below, protecting groups for sensitive or reactive groups are employed where necessary in accordance with general principles of synthetic chemistry. Protecting groups are manipulated according to standard methods of organic synthesis (T. W. Green and P. G. M. Wuts (1991) Protecting Groups in Organic Synthesis, John Wiley & Sons, with regard to protecting groups). These groups are removed at a convenient stage of the compound synthesis using methods that are readily apparent to those skilled in the art. The selection of processes as well as the reaction conditions and order of their execution shall be consistent with the preparation of compounds of the present invention.

Those skilled in the art will recognize if a stereocenter exists in compounds of the present invention. Accordingly, the present invention includes all possible stereoisomers and includes not only racemic compounds but the individual enantiomers as well. When a compound is desired as a single enantiomer, such may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994), with regard to stereochemistry.

Representative LXR modulators according to the current invention include:

1,1,1,3,3,3-hexafluoro-2-{4-[methyl(phenylmethyl)amino]phenyl}-2-propanol;
2-{4-[Butyl(□yridine-2-ylmethyl)amino]phenyl}-1,1,1,3,3,3-hexafluoropropan-2-ol;
2-{4-[Benzyl(butyl)amino]phenyl}-1,1,1,3,3,3-hexafluoropropan-2-ol;
2-(4-{Butyl[3-(2-hydroxyethoxy)benzyl]amino}phenyl)-1,1,1,3,3,3-hexafluoropropan-2-ol ;
2-{4-[Butyl(pyridin-4-ylmethyl)amino]phenyl}-1,1,1,3,3,3-hexafluoropropan-2-ol;
3-[(Butyl{4-[2,2,2-trifluoro-1-hydroxy-1-(trifluoromethyl)ethyl]phenyl}amino)methyl]phenol;
2-[(Butyl{4-[2,2,2-trifluoro-1-hydroxy-1-(trifluoromethyl)ethyl]phenyl}amino)methyl]phenol;
4-[(Butyl{4-[2,2,2-trifluoro-1-hydroxy-1-(trifluoromethyl)ethyl]phenyl}amino)methyl]phenol;
2-[(Butyl{4-[2,2,2-trifluoro-1-hydroxy-1-(trifluoromethyl)ethyl]phenyl}amino)methyl]-6-fluorophenol;
2-[(Butyl{4-[2,2,2-trifluoro-1-hydroxy-1-(trifluoromethyl)ethyl]phenyl}amino)methyl]-4-(trifluoromethoxy)phenol;
2-[(Butyl{4-[2,2,2-trifluoro-1-hydroxy-1-(trifluoromethyl)ethyl]phenyl}amino)methyl]-4,6-dichlorophenol;
2-[(Butyl{4-[2,2,2-trifluoro-1-hydroxy-1-(trifluoromethyl)ethyl]phenyl}amino)methyl]-4-chlorophenol;
4-[(Butyl{4-[2,2,2-trifluoro-1-hydroxy-1-(trifluoromethyl)ethyl]phenyl}amino)methyl]-2-chlorophenol;
4-[(Butyl{4-[2,2,2-trifluoro-1-hydroxy-1-(trifluoromethyl)ethyl]phenyl}amino)methyl]-2-chloro-6-methoxyphenol;
4-[(Butyl{4-[2,2,2-trifluoro-1-hydroxy-1-(trifluoromethyl)ethyl]phenyl}amino)methyl]-2,6-dichlorophenol;
2-[4-(butyl{[3-chloro-4,5-bis(methyloxy)phenyl]methyl}amino)phenyl]-1,1,1,3,3,3-hexafluoro-2-propanol;
2-{4-[Butyl(3,5-dichlorobenzyl)amino]phenyl}-1,1,1,3,3,3-hexafluoropropan-2-ol;
2-{4-[Butyl(3,5-dichloro-4-methoxybenzyl)amino]phenyl}-1,1,1,3,3,3-hexafluoropropan-2-ol, or a salt or solvate thereof.

ABBREVIATIONS

As used herein the symbols and conventions used in these processes, schemes and examples are consistent with those used in the contemporary scientific literature, for example, the *Journal of the American Chemical Society* or the *Journal of Biological Chemistry.* Specifically, the following abbreviations may be used in the examples and throughout the specification:

| | |
|---|---|
| g (grams); | mg (milligrams); |
| L (liters); | mL (milliliters); |
| μL (microliters); | psi (pounds per square inch); |
| M (molar); | mM (millimolar); |
| Hz (Hertz); | MHz (megahertz); |
| mol (moles); | mmol (millimoles); |
| rt (room temperature); | min (minute); |
| h (hour); | mp (melting point); |
| TLC (thin layer chromatography); | $CDCl_3$ (deuterated chloroform); |
| $CD_3OD$ (deuterated methanol); | $SiO_2$ (silica); |
| DMSO (dimethylsulfoxide); | EtOAc (ethyl acetate); |
| atm (atmosphere); | HCl (hydrochloric acid); |
| $CHCl_3$ (chloroform) ; | DMF (*N*,*N*-dimethylformamide); |
| Ac (acetyl); | Me (methyl) ; |
| Et (ethyl) ; | EtOH (ethanol) ; |
| MeOH (methanol); | t-Bu (tert-butyl); |
| $Et_2O$ (diethyl ether); | $N_2$ (nitrogen); |
| MsCl (methanesulphonyl chloride); | sat'd (saturated); |
| DMAP (4-(dimethylamino)pyridine); | AcHO (acetic acid); |
| DCM (dichloromethane); | LAH (Lithium Aluminum Hydride); |
| IPTG (isopropyl-beta-D-thiogalactopyranoside); | |
| TBS (tris buffered saline); | DTT (dithiothreitol); |
| EDTA (ethylenediaminetetraacetic acid); | |
| BSA (bovine serum albumin); | SPA (scintillation proximity assay); |
| ELISA (enzyme-linked immunosorbant assay). | |

Unless otherwise indicated, all temperatures are expressed in °C (degrees Centigrade). All reactions conducted under an inert atmosphere at room temperature unless otherwise noted. Reagents employed without synthetic details are commercially available or made according to literature procedures.

$^{1}$H-NMR spectra were recorded on a Varian Gemini 400 MHz NMR spectrometer. $^{1}$H-NMR spectra are reported as chemical shift δ, number of protons, multiplicity (s, singlet; d, doublet; t, triplet; m, multiplet; br s, broad singlet) and coupling constant (J) in Hertz. Electron Spray (ES) or Chemical Ionization (CI) was recorded on a Hewlett Packard 5989A mass spectrometer.

**SCHEMES**

**Scheme I**

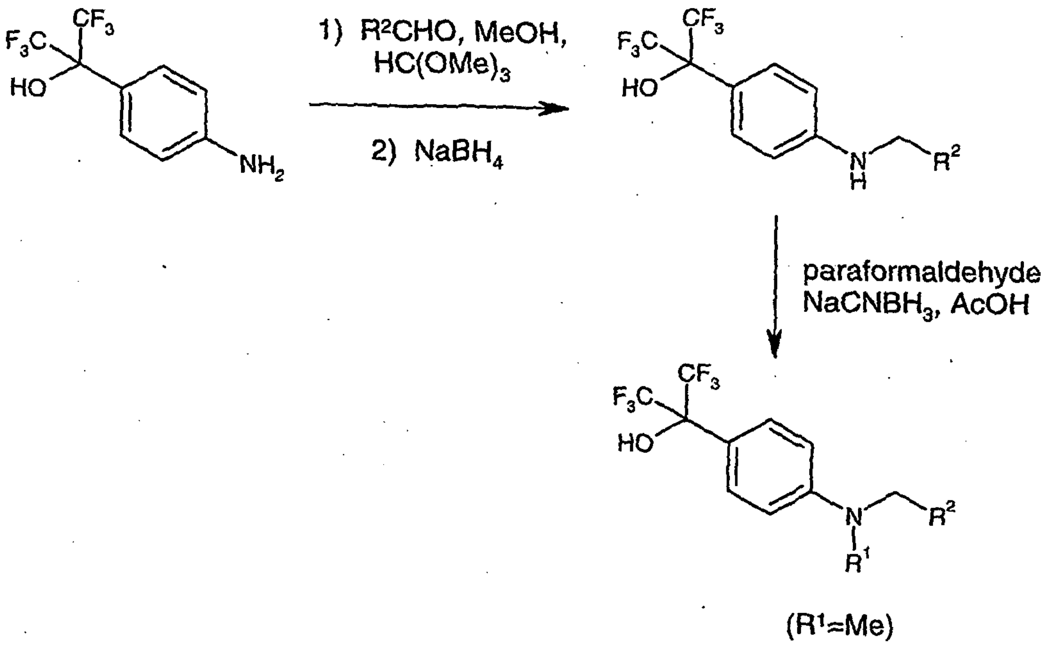


**Scheme II**

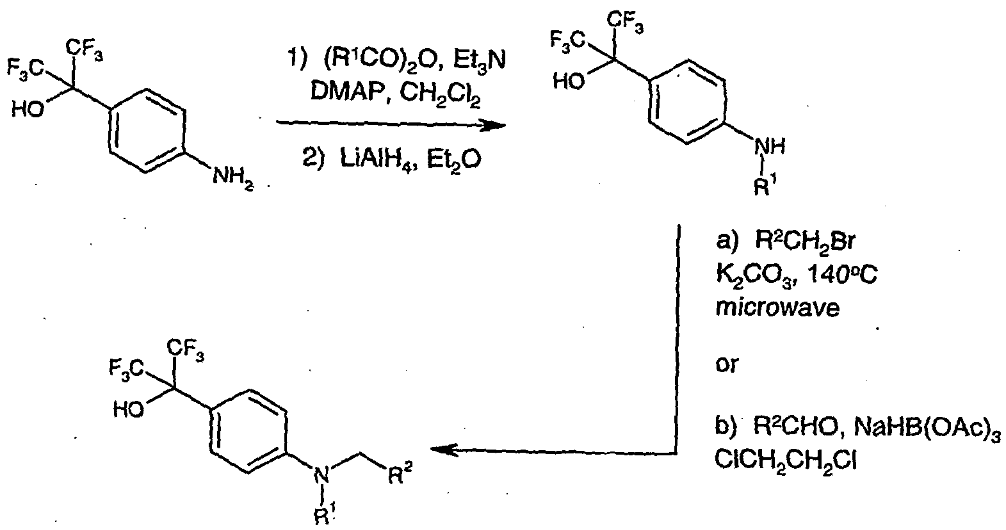


**EXAMPLES**

**Intermediate 1:** *2-[4-(butylamino)phenyl]-1,1,1,3,3,3-hexafluoro-2-propanol*

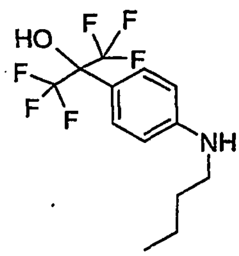

To a solution of 10g (0.04 mol) of 2-(4-aminophenyl)-1,1,1,3,3,3-hexafluoropropan-2-ol in 170 mL of DCM was added 12 mL (0.07 mol) butyric anhydride and 14 mL (0.09 mol) triethylamine in the presence of a catalytic amount of DMAP. The mixture was heated at 40°C for 18 hours. After this time, the reaction mixture was evaporated to dryness. The residue was dissolved in ethyl acetate and added with 10% potassium carbonate solution. The mixture was stirred at room temperature for 2h. At this time, a solid was precipitated. The solid was collected by filtration as the crude butyric amide/ester product. This amide/ester was treated with 95 mL 1 M LAH in diethyl ether at room temperature for 4h. At this time, 20mL water was added slowly at 0°C, followed by addition of 50 mL aqueous 15% sodium hydroxide. The resulting mixture was filtered through Celite. The filtrate was extracted with ethyl acetate, dried over anhydrous magnesium sulfate, and evaporated to dryness to provide 9.5g of Intermediate 1. $^1$H-NMR ($CDCl_3$) δ 0.98 (t, 3H), 1.45 (m, 2H), 1.63 (m, 2H), 3.15 (t, 2H), 6.63 (dd, J=1.9, 9.0 Hz, 2H), 7.45 (d, J=8.7 Hz, 2H).

**Example 1:** *1,1,1,3,3,3-hexafluoro-2-{4-[methyl(phenylmethyl)amino]phenyl}-2-propanol*

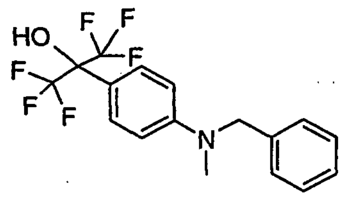

A solution of 101 mg (0.39 mmol) of 2-(4-aminophenyl)-1,1,1,3,3,3-hexafluoro-2-propanol in 800 μl of MeOH/trimethyl orthoformate (1:1) at room temperature was treated with 40 μL (0.39 mmol) of benzaldehyde. After stirring overnight, the reaction was treated with solid sodium borohydride in small portions until TLC indicated the intermediate imine was consumed. The reaction was filtered through a pad of silica gel using EtOAc as eluent, and the filtrate was concentrated in vacuo. Purification by silica gel chromatography (4:1/hexanes:EtOAc) provided 85 mg (63%) of an intermediate secondary amine. A solution of 60 mg (0.17 mmol) this intermediate in 600 μL of glacial acetic acid was treated with excess paraformaldehyde followed by 53 mg (0.85 μmol) of $NaCNBH_3$. After stirring at room temperature for 15 h, the reaction was filtered through silica gel using EtOAc as eluent, and the filtrate was concentrated in vacuo. Purification by preparative TLC (silica gel, 1000 μm) using 4:1/hexanes:EtOAc provide 30 mg (50%) of the title compound: $^1$H-NMR ($CDCl_3$) δ 3.07 (s, 3H), 4.56 (s, 2H), 6.76 (d, J= 8.8 Hz, 2H), 7.18-7.29 (m, 3H), 7.33 (t, J=7.4 Hz , 2H), 7.49 (d, J=8.8 Hz, 2H). Mass Spectrum (ES) m/e= 364 (M+1).

**Example 2.** *2-{4-[Butyl(pyridine-2-ylmethyl)amino]phenyl}-1,1,1,3,3,3-hexafluoropropan-2-ol*

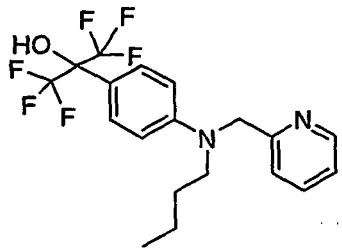

To a solution of 0.06g (0.32 mmol) 2-(bromomethyl)pyridine in 0.2 mL DMF was added 0.05 g (0.16 mmol) 2-[4-(butylamino)phenyl]-1,1,1,3,3,3-hexafluoropropan-2-ol (intermediate 1) and 0.06 g (0.48 mmol) potassium carbonate. The mixture was microwaved at 140°C for 15 minutes. After this time, the reaction mixture was evaporated to dryness. The residue was purified on prep Agilent HPLC system with a Phenomenex Luna 5μ micro C18 (150x21mm) column and eluted with 30% to 100% acetonitrile in water in the presence of 0.1 % trifluoroacetic acid over 10 minutes to give the title compound: $^1$H-NMR ($CD_3OD$) δ 1.01 (t, 3H), 1.45 (m, 2H), 1.71 (m, 2H), 3.61 (t, 2H), 4.98 (s, 2H), 6.79 (d, J= 9.1 Hz, 2H), 7.54 (d, J= 8.8 Hz, 2H), 7.86 (t, 2H), 8.42 (t, 1H), 8.72 ( d, J=5.5Hz, 1H). Mass Spectrum (APCI) m/e= 407 (M+1).

**Example 3.** *2-{4-[Benzyl(butyl)amino]phenyl}-1,1,1,3,3,3-hexafluoropropan-2-ol*

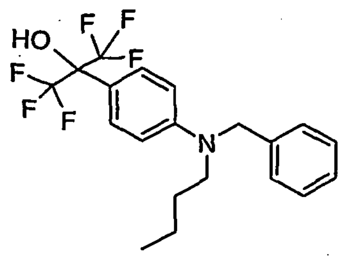

To a solution of 1.7g (15.85 mmol) of benzaldehyde in 30 mL of DCE was added 1 mL glacial acetic acid, 1g (3.2 mmol) 2-[4-(butylamino)phenyl]-1,1,1,3,3,3-hexafluoropropan-2-ol (Intermediate 1) and 3.4g (16 mmol) sodium triacetoxyborohydride. The mixture was stirred at room temperature for 18 hours. After this time, the reaction mixture was evaporated to dryness. The residue was purified on prep Agilent HPLC system with a Phenomenex Luna 5μ C18 (150x21 mm) column and eluted with 30% to 100% acetonitrile in water in the presence of 0.1 % trifluoroacetic acid over 10 minutes to give the title compound: $^1$H-NMR ($CD_3OD$) δ0.97 (t, 3H), 1.41 (m, 2H), 1.65 (m, 2H), 3.49 (t, 2H), 4.62 (s, 2H), 6.76 (d, J= 9.0 Hz, 2H), 7.21-7.33 (m, 5H), 7.48 (d, J=8.8 Hz, 2H). Mass Spectrum (APCI) m/e= 406 (M+1).

The following compounds were prepared in a similar fashion:

**Example 4.** *2-(4-{Butyl[3-(2-hydroxyethoxy)benzyl]amino}phenyl)-1,1,1,3,3,3-hexafluoropropan-2-ol*

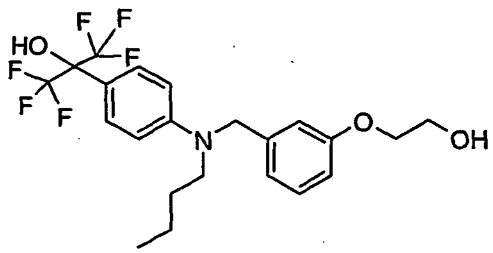

$^1$H-NMR ($CD_3OD$) δ 0.98 (t, 3H), 1.42 (m, 2H), 1.66 (m, 2H), 3.48 (t, 2H), 3.84 (t, 2H), 3.98 (t, 3H), 4.58 (s, 2H), 6.72 (d, J = 9.0 Hz, 2H), 6.81 (s, 1H), 6.82 ( d, J=6.9 Hz, 2H), 7.23 (t, 1H), 7.45 (d, J= 8.7 Hz, 2H). Mass Spectrum (APCI)

m/e= 466 (M+1).

**Example 5.** *2-{4-(Bufyl(pyridin-4-ylmethyl)amino]phenyl}-1,1,1,3,3,3-hexafluoropropan-2-ol*

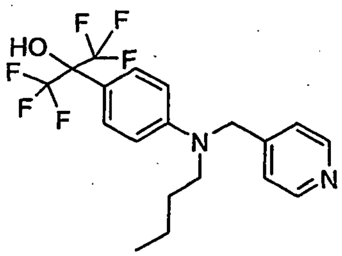

$^1$H-NMR ($CD_3OD$) δ 1.01 (t, 3H), 1.46 (m, 2H), 1.70 (m, 2H), 3.59 (t, 2H), 4.94 (s, 2H), 6.72 (d, J= 9.2 Hz, 2H), 7.50 (d, J=8.7 Hz, 2H), 7.90 (d, J= 4.8 Hz, 2H), 8.74 (br s, 2H), Mass Spectrum (APCI) m/e= 407 (M+1).

**Example 6.** *3-[(Butyl{4-[2,2,2-trifluoro-1-hydroxy-1-(trifluoromethyl)ethyl]phenyl}amino)methyl]phenol*

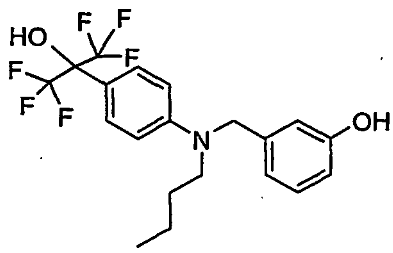

$^1$H-NMR ($CD_3OD$) δ 0.98 (t, 3H), 1.41 (m, 2H), 1.67 (m, 2H), 3.45 (t, 2H), 4.54 (s, 2H), 6.65-6.73 (m, 5H), 7.14 (m, 1H), 7.45 (d, J= 8.9 Hz, 2H). Mass Spectrum (APCI) m/e= 422 (M+1).

**Example 7.** *2-[(Butyl{4-[2,2,2-trifluoro-1-hydroxy-1-(trifluoromethyl)ethyl]phenyl}amino)methyl]phenol*

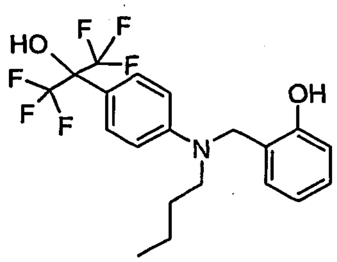

$^1$H-NMR ($CDCl_3$) δ 0.91 (t, 3H), 1.32 (m, 2H), 1.56 (m, 2H), 3.31 (t, 2H), 4.47 (s, 2H), 6.87 (m, 2H), 7.03 (d, J=8.9 Hz, 2H), 7.18 (m, 2H), 7.59 (d, J=8.7 Hz, 2H). Mass Spectrum (APCI) m/e= 422 (M+1).

**Example 8.** *4-[(Butyl{4-[2,2,2-trifluoro-1-hydroxy-1-(trifluoromethyl)ethyl]phenyl}amino)methyl]phenol*

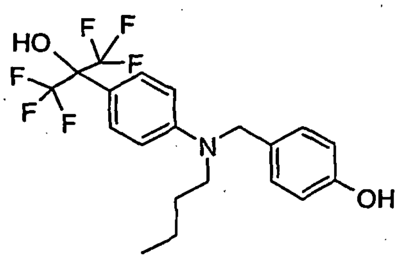

$^1$H-NMR ($CD_3OD$) δ 0.89 (t, 3H), 1.25 (m, 2H), 1.51 (m, 2H), 3.08 (t, 2H), 3.88 (s, 2H), 6.73 (d, J= 8.4 Hz, 2H), 6.84 (d, J= 8.7 Hz, 1H), 6.99 (d, J=8.4 Hz, 2H), 7.43 (s, 1H), 7.52(d, J=8.6 Hz, 1H). Mass Spectrum (ES) m/e= 422 (M+1).

**Example 9.** *2-[(Butyl{4-[2,2,2-trifluoro-1-hydroxy-1-(trifluoromethyl)ethyl]phenyl}amino)methyl]-6-fluorophenol*

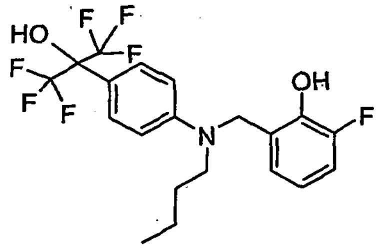

$^1$H-NMR ($CD_3OD$) δ 0.98 (t, 3H), 1.43 (m, 2H), 1.67 (m, 2H), 3.50 (t, 2H), 4.61 (s, 2H), 6.78 (m, 4H), 6.96 (m, 1H), 7.48 (d, J=8.8 Hz, 2H). Mass Spectrum (APCI) m/e= 440 (M+1).

**Example 10.** *2-[(Butyl{4-[2,2,2-trifluoro-1-hydroxy-1-(trifluoromethyl)ethyl]phenyl}amino)methyl]-4-(trifluoromethoxy) phenol*

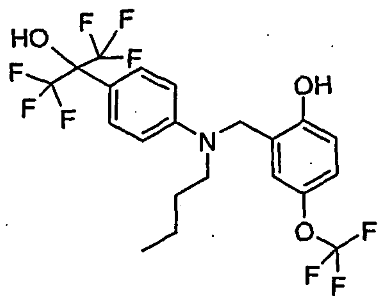

$^1$H-NMR ($CD_3OD$) δ 0.98 (t, 3H), 1.42 (m, 2H), 1.67 (m, 2H), 3.50 (t, 2H), 4.57 (s, 2H), 6.74-6.88 (m, 4H), 6.98 (dd, J=2.2, 8.6 Hz, 1H), 7.50 (d, J=8.7 Hz, 2H). Mass Spectrum (ES) m/e= 506 (M+1).

**Example 11.** *2-[(Butyl{4-[2,2,2-trifluoro-1-hydroxy-1-(trifluoromethyl)ethyl]phenyl}amino)methyl]-4,6-dichlorophenol*

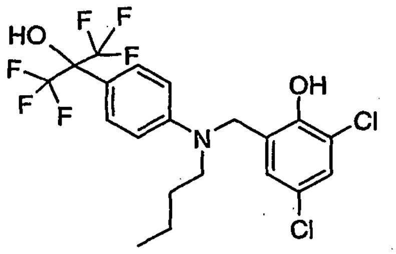

$^1$H-NMR ($CD_3OD$) δ 0.90 (t, 3H), 1.41 (m, 2H), 1.68 (m, 2H), 3.48 (t, 2H), 4.57 (s, 2H), 6.70 (d, J=9.0 Hz, 2H), 6.86 (d, J=2.3 Hz, 1H), 7.25 (d, J=2.5 Hz, 1H), 7.49 (d, J=8.7 Hz, 2H). Mass Spectrum (APCI) m/e= 490 (M+1).

**Example 12.** *2-[(Butyl{4-[2,2,2-trifluoro-1-hydroxy-1-(trifluoromethyl)ethyl]phenyl}amino)methyl]-4-chlorophenol*

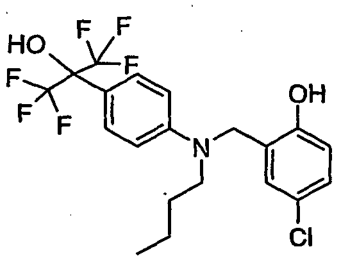

$^1$H-NMR ($CD_3OD$) δ 0.99 (t, 3H), 1.43 (m, 2H), 1.66 (m, 2H), 3.52 (t, 2H), 4.56 (s, 2H), 6.77-6.9 0 (m, 4H), 7.06(dd, J=2.6, 8.6 Hz, 1H), 7.54 (d, J=8.9 Hz, 2H). Mass Spectrum (ES) m/e= 456 (M+1).

**Example 13.** *4-[(Butyl{4-[2,2,2-trifluoro-1-hydroxy-1-(trifluoromethyl)ethyl]phenyl}amino)methyl]-2-chlorophenol*

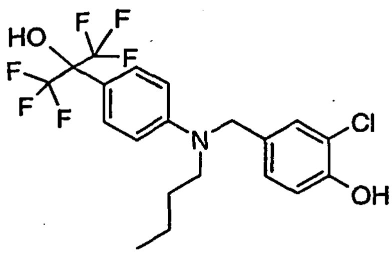

$^1$H-NMR ($CD_3OD$) δ 0.96 (t, 3H), 1.39 (m, 2H), 1.61 (m, 2H), 3.50 (t, 2H), 4.53 (s, 2H), 6.83-6.99 (m, 4H), 7.13(d, J=1.9 Hz, 1H), 7.55 (d, J=8.7 Hz, 2H). Mass Spectrum (ES) m/e= 456 (M+1).

**Example 14.** *4-[(Butyl{4-[2,2,2-trifluoro-1-hydroxy-1-(trifluoromethyl)ethyl]phenyl}amino)methyl]-2-chloro-6-methoxy-phenol*

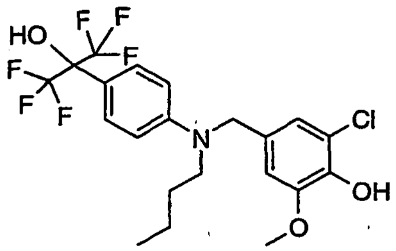

$^1$H-NMR ($CD_3OD$) δ 0.96 (t, 3H), 1.41 (m, 2H), 1.61 (m, 2H), 3.51 (t, 2H), 3.77 (s, 3H), 4.53 (s, 2H), 6.69 (d, J= 1.7 Hz, 1H), 6.76 (d, J= 1.6 Hz, 1H), 6.89 (d, J= 9.0 Hz, 2H), 7.56 (d, J=8.7 Hz, 2H). Mass Spectrum (ES) m/e= 486 (M+1).

Example 15. *4-[(Butyl{4-[2,2,2-trifluoro-1-hydroxy-1-(trifluoromethyl)ethyl]phenyl}amino)methyl]-2,6-dichlorophenol*

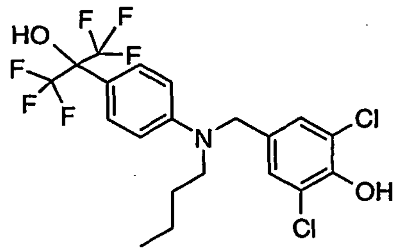

$^1$H-NMR ($CD_3OD$) δ 0.98 (t, 3H), 1.41 (m, 2H), 1.64 (m, 2H), 3.46 (t, 2H), 4.50 (s, 2H), 6.75 (d, J= 9.1 Hz, 2H), 7.12 (s, 2H), 7.50 (d, J= 8.9 Hz,2H). Mass Spectrum (APCI) m/e= 490 (M+1).

**Example 16.** *2-[4-(butyl{[3-chloro-4,5-bis(methyloxy)phenyl]methyl}amino)phenyl]-1,1,1,3,3,3-hexafluoro-2-propanol*

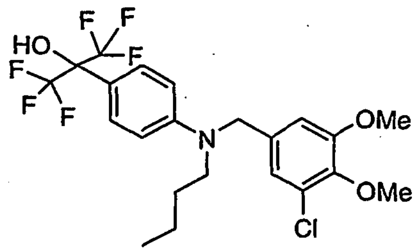

$^1$H-NMR ($CD_3OD$) δ 0.98 (t, J=7.2 Hz, 3H), 1.30-1.50 (m, 2H), 1.55-1.75 (m, 2H), 3.50 (t, J=7.7 Hz, 2H), 3.78 (s, 3H), 3.79 (s, 3H), 4.56 (s, 2H), 6.82 (dd, J = 6.3, 1.7 Hz, 4H), 7.54 (d, J = 8.8 Hz, 2H); Mass Spectrum (ES) *m*/*e* = 500 (M+1).

**Example 17.** *2-{4-[Butyl(3,5-dichlorobenzyl)amino]phenyl}-1,1,1,3,3,3-hexafluoropropan-2-ol*

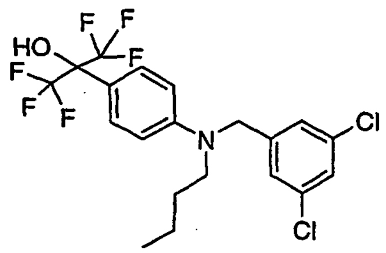

$^{1}$H-NMR ($CD_3OD$) δ 0.98 (t, 3H), 1.42 (m, 2H), 1.66 (m, 2H), 2.51 (s, 3H), 3.48 (t, 2H), 4.55 (s, 2H), 6.71 (d, J = 9.1 Hz, 2H), 7.17 (d, J= 1.5 Hz, 2H), 7.30 (s, 1H), 7.49 (d, J= 8.9 Hz, 2H). Mass Spectrum (APCI) m/e= 474 (M+1).

**Example 18.** *2-{4-[Butyl(3,5-dichloro-4-methoxybenzyl)amino]phenyl}-1,1,1,3,3,3-hexafluoropropan-2-ol*

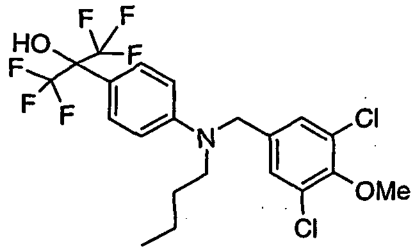

$^{1}$H-NMR ($CD_3OD$) δ 0.98 (t, 3H), 1.41 (m, 2H), 1.64 (m, 2H), 3.48 (t, 2H), 3.85 (s, 3H), 4.55 (s, 2H), 6.74 (d, J = 9.1 Hz, 2H), 7.21 (s, 2H), 7.52 (d, J= 8.8 Hz, 2H). Mass Spectrum (ES) m/e= 504 (M+1).

BIOLOGICAL SECTION

Compounds of the current invention are modulators of LXRβ. Additionally, the compounds of the present invention may also prove useful as modulators of LXRα. Activity mediated through the LXRs was determined using the following assays.

**LXRα and β binding assays:**

**LXR BETA**

Human LXRβ ligand binding domain (LXRβ LBD) was expressed in E.coli strain BL21(DE3) as an amino-terminal polyhistidine tagged fusion protein. Expression was under the control of an IPTG inducible T7 promoter. DNA encoding this recombinant protein and a modified polyhistidine tag was subcloned into the expression vector pRSETa (Invitrogen). The sequence of the modified polyhistidine tag (MKKGHHHHHHG) was fused in frame to residues 185-461 of LXRβ. The coding sequence of Human LXRβ LBD was derived from Genbank accession number U 07132 (BioResources # 5464). The resulting complete encoded sequence is as follows: MKKGHHHHHHGPVGPQGSSSSASGPGASPGGSEA-GSQGSGEGEGVQLTAAQELMI QQLVAAQLQCNKRSFSDQPKVTPWPLGADPQSRDARQQRFAHFTELAIISVQEIV-DFA KQVPGFLQLGREDQIALLKASTIEIMLLETARRYNHETECITFLKDFTYSKDDFHRAGLQ VEFINPIFEFSRAMRR-LGLDDAEYALLIAINIFSADRPNVQEPGRVEALQQPYVEALLSY TRIKRPQDQLRFPRMLMKLVSLRTLSSVHSEQVF-ALRLQDKKLPPLLSEIWDVHE. Ten-liter fermentation batches were grown in Rich $PO_4$ media with 0.1 mg/mL Ampicillin at 25°C for 12 hours, cooled to 9°C and held at that temperature for 36 hours to a density of $OD_{600}$=14. At this cell density, 0.25 mM IPTG was added and induction proceeded for 24 hours at 9°C, to a final $OD_{600}$ = 16. Cells were harvested by centrifugation (20 minutes, 3500 g, 4°C), and concentrated cell slurries were stored in PBS at -80°C.

**Purification of LXRβ Ligand Binding Domain:** 30-40 g cell paste (equivalent to 2-3 liters of the fermentation batch) was resuspended in 300-400 mL TBS, pH 8.5 (2 5mM Tris, 150 mM NaCl). Cells were lysed by passing 3 times through a homogenizer (Rannie) and cell debris was removed by centrifugation (30 minutes, 20,000 g, 4°C). The cleared supernatant was filtered through coarse pre-filters, and TBS, pH 8.5, containing 500 mM imidazole was added to obtain a final imidazole concentration of 50 mM. This lysate was loaded onto a column (6 x 8 cm) packed with Sepharose [Ni++ charged] chelation resin (Pharmacia) and pre-equilibrated with TBS pH 8.5/ 50mM imidazole. After washing to baseline absorbance with equilibration buffer, the column was developed with a linear gradient of 50 to 275 mM imidazole in TBS, pH 8.5. Column fractions were pooled and dialyzed against TBS, pH 8.5, containing 5% 1,2-propanediol, 5 mM

DTT and 0.5 mM EDTA. The protein sample was concentrated using Centri-prep 10K (Amicon) and subjected to size exclusion, using a column (3 x 90 cm) packed with Sepharose S-75 resin (Pharmacia) pre-equilibrated with TBS, pH 8.5, containing 5 % 1,2-propanediol, 5mM DTT and 0.5mM EDTA.

**Biotinylation of LXRβ:** Purified LXRβ LBD was desalted/ buffer exchanged using PD-10 gel filtration columns into PBS [100 mM sodium phosphate, pH 7.2, 150 mM NaCl]. LXRβ LBD was diluted to approximately 10 mM in PBS and five-fold molar excess of NHS-LC-Biotin (Pierce) was added in a minimal volume of PBS. This solution was incubated with gentle mixing for 30 minutes at ambient room temperature. The biotinylation modification reaction was stopped by the addition of 2000x molar excess of Tris-HCl, pH 8. The modified LXRβ LBD was dialyzed against 4 buffer changes, each of at least 50 volumes, PBS containing 5 mM DTT, 2 mM EDTA and 2% sucrose. The biotinylated LXRβ LBD was subjected to mass spectrometric analysis to reveal the extent of modification by the biotinylation reagent. In general, approximately 95% of the protein had at least a single site of biotinylation, and the overall extent of biotinylation followed a normal distribution of multiple sites, ranging from one to nine

**Assay Buffer for LXRβ SPA:** 50 mM MOPS pH 7.5, 50 mM NaF, 0.05 mM CHAPS. 0.1 mg/ml Fraction 5 fatty acid free BSA. Add solid DTT to assay buffer immediately prior to use in assay buffer (final concentration = 10 mM).

**Preparation of a SPA bead/LXRβ/$^3$H-radioligand solution:** To buffer containing 10 mM of freshly added DTT from solid, add an appropriate amount of 5 mg/ml SPA bead solution to a final concentration of 0.25 mg/ml. Add an appropriate amount of LXRβ LBD to give a final concentration of 25 nM and invert gently to mix. Incubate 30 minutes at room temperature, spin down beads at 2500 rpm for 10 minutes, and carefully remove supernatant without disturbing the bead pellet. Dilute to the original volume with assay buffer containing 10 mM of freshly added DTT. Add radiolabeled ligand (*N*-$^3H_3$-methyl-*N*-{4-[2,2,2-trifluoro-1-hydroxy-1-(trifluoromethyl)ethyl]phenyl}benzenesulfonamide) to the bead solution to a final concentration of 10 nM. Using a multidrop add 100 ul to each well of a 96-well plate containing test compounds, incubate 30 minutes at room temperature and read on Microbeta 1450 Trilux

Normalize the data by the following. (1-(unknown- average non-specific)/ (average 100%- average non-specific)) * 100 = % inhibition

**LXR ALPHA**

Human LXRα Ligand Binding Domain (LXRα LBD) was expressed in E.coli strain BL21(DE3) as an amino-terminal polyhistidine tagged fusion protein. Expression was under the control of an IPTG inducible T7 promoter. DNA encoding this recombinant protein was subcloned into the pRSETa expression vector (Invitrogen). Sequence encoding amino acids 183-447 of human LXRα was fused in frame to the polyhistidine tag derived from the vector (MRGSHHHHHHGMAS) or to a modified histidine tag (MKKGHHHHHHG). The coding sequence of human LXRα LBD was derived from Genbank accession number U22662 (BioResources # 18711 and # 13635). The resulting complete encoded sequence is as follows: MKKGHHHHHHGEEEQAHATSLPPRASSPPQILPQLSPEQLGMIEKLVAAQQQCNRRS FSDRLRVTP-WPMAPDPHSREARQQRFAHFTELAIVSVQEIVDFAKQLPGFLQLSRED QIALLKTSAIEVMLLETSRRYNPGSESITF-LKDFSYNREDFAKAGLQVEFINPIFEFSRA MNELQLNDAEFALLIAISIFSADRPNVQDQLQVERLQHTYVEALHAYV-SIHHPHDRLMF PRMLMKLVSLRTLSSVHSEQVFALRLQDKKLPPLLSEIWDVHE. Ten-liter fermentation batches were grown in Rich $PO^4$ media with 0.1 mg/mL ampicillin at 25°C for 12 hours, cooled to 9°C and held at that temperature for 36 hours to a density of $OD_{600}$=14. At this cell density, 0.25 mM IPTG was added and induction proceeded for 24 hours at 9°C, to a final $OD_{600}$ = 16. Cells were harvested by centrifugation (20 minutes, 3500 g, 4°C), and concentrated cell slurries were stored in PBS at -80°C.

**Purification of LXRα Ligand Binding Domain:** Typically 50-100 g of cell paste is resuspended in 250-750 mL TBS, pH 8.5 (25mM Tris, 150 mM NaCl). Cells are lysed by passing 3 times through an APV Rannie MINI-lab homogenizer and cell debris is removed by centrifugation (30 minutes, 20,000g, 4°C). The cleared supernatant is filtered through coarse pre-filters, and TBS, pH 8.5, containing 500 mM imidazole is added to obtain a final imidazole concentration of 50 mM. This lysate is loaded onto a column (XK-26, 10 cm) packed with Sepharose [Ni++ charged] Chelation resin (Pharmacia) and pre-equilibrated with TBS pH 8.5/ 50 mM imidazole. After washing to baseline absorbance with equilibration buffer, the column is washed with approximately one column volume of TBS pH -8.5 containing 90 mM imidazole. LXRα LBD(183-447) is eluted with a gradient from 50 to 500 mM imidazole. Column peak fractions are pooled immediately and diluted 4-5 fold with 25 mM Tris pH 8.5, containing 5 % 1,2-propanediol, 0.5 mM EDTA and 5 mM DTT. The diluted protein sample is then loaded onto a column (XK-16, 10cm) packed with Poros HQ resin (anion exchange). After washing to baseline absorbance with the dilution buffer the protein is eluted with a gradient from 30 -500 mM NaCl. Peak fractions are pooled and concentrated using Centri-prep 10K (Amicon) and subjected to size exclusion, using a column (XK-26, 90 cm) packed with Superdex-75 resin (Pharmacia) pre-equilibrated with TBS, pH 8.5, containing 5 % 1,2-propanediol, 0.5 mM EDTA and 5 mM DTT.

**Biotinylation of LXRα:** Purified LXRα LBD was desalted/ buffer exchanged using PD-10 gel filtration columns into

PBS [100 mM NaPhosphate, pH 8.5, 150 mM NaCl]. LXRα LBD was diluted to approximately 10 mM in PBS and five-fold molar excess of NHS-LC-Biotin (Pierce) was added in a minimal volume of PBS. This solution was incubated with gentle mixing for 30 to 60 minutes at ambient temperature. The biotinylation modification reaction was stopped by the addition of 2000x molar excess of Tris-HCl, pH 8. The modified LXRα LBD was dialyzed against 4 buffer changes, each of at least 50 volumes, of 25 mM Tris pH 8.5 containing 150 mM NaCl, 5mM DTT, 2 mM EDTA and 2% sucrose. The biotinylated LXRα LBD was subjected to mass spectrometric analysis to reveal the extent of modification by the biotinylation reagent. In general, approximately 95% of the protein had at least a single site of biotinylation, and the overall extent of biotinylation followed a normal distribution of multiple sites, ranging from one to six.

**Assay Buffer for LXRα SPA:** 50 mM MOPS pH 7.5, 50 mM NaF, 0.05 mM CHAPS. 0.1mg/ml Fraction 5 fatty acid free BSA. Add solid DTT to assay buffer immediately prior to use in assay buffer (final concentration =10 mM).

**Preparation of a working SPA bead/LXRα/$^3$H-radioligand solution:** To buffer containing 10 mM of freshly added DTT from solid, add an appropriate amount of 5 mg/ml SPA bead solution to a final concentration of 0.25 mg/ml. Add an appropriate amount of LXRα LBD to give a final concentration of 25 nM and invert gently to mix. Incubate 30 minutes at room temperature. Spin down beads at 2500 rpm for 10 minutes Carefully remove supernatant without disturbing the bead pellet. Dilute to the original volume with assay buffer containing 10 mM of freshly added DTT. Add radioligand (*N*-$^3H_3$-methyl-*N*-{4-[2,2,2-trifluoro-1-hydroxy-1-(trifluoromethyl)ethyl]phenyl}benzenesulfonamide) to the bead solution to a final concentration of 10 nM. Using a multidrop add 100 ul to each well of a 96-well plate containing test compounds, incubate 30 minutes at room temperature and read on Microbeta 1450 Trilux

Normalize the data by the following. (1-(unknown- average non-specific)/ (average 100%- average non-specific)) * 100 = % inhibition

| Example Numbed | IC50 (nM) in LXRβ binding assay (as described above) | IC50 (nM) in LXRα binding assay (as described above) |
|---|---|---|
| 1-18 | ≤ 750 | ≤ 1000 |

**IL-6 ELISA:** The human monocyte/macrophage cell line, THP-1 (ATCC, Manassas, VA.), was differentiated with 40ng/mL 1α,25-dihydroxy-Vitamin $D_3$-(EMD Biosciences, Inc., San Diego, CA) at 10x10$^6$ cells/50mL standard cell culture media for 72 hours. Differentiated THP-1 cells were seeded in 96 well plates (1 x 10$^5$/ml, 200 μl/well) in complete media. Compounds in DMSO solution (0.2% DMSO final) were added to duplicate wells, with final concentrations ranging from 2.3 nM to 5.0 μM (37°C/5% $CO_2$ humidified incubator). After a 6 hour pretreatment with compounds or vehicle, lipopolysaccharides (Sigma, St. Louis, MO) were added to each well to a final concentration of 100ng/mL. Plates were then incubated for an additional 18 hours (37°C/5% $CO_2$ humidified incubator). Plates were centrifuged at 1200 rpm in tabletop centrifuge for 5 minutes to pellet all cells. Media was removed and transferred to separate 96-well plate and stored at 4°C until assayed. Human IL-6 in the media was assayed using IL-6 ELISA kit (R&D Systems, Minneapolis, MN). Treatment of THP-1 cells with compounds of the present invention reduce the amount of IL-6 found in the media, as measured in the above described assay, when compared to vehicle treated cells. Compounds with an IC50 less than 100nM are preferred.

**Triglyceride accumulation assay:** HepG2 cells were seeded in 96 well plates (1 x 10$^5$/ml, 200 μl/well) in complete media. Twenty four hours later, compounds in DMSO solution were added to triplicate wells, with final concentrations ranging from 2.3 nM to 5 μM. After 4d at 37°C in a 5% $CO_2$ humidified incubator, cells were lysed in 50 ul 0.1% NP-40 per well. Cellular triglycerides were measured using a triglyceride (GPO) reagent set (ThermoDMA, Arlington, TX) as per the manufacturer's protocol. Compounds that exhibit little or no triglyceride accumulation, as compared to vehicle treated cells, are preferred.

## Claims

1. A compound of formula (I):

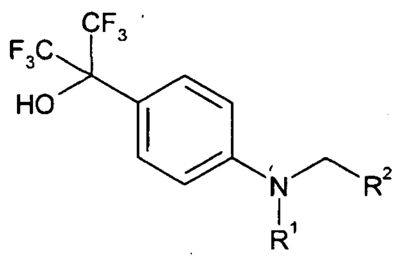

(I)

or a salt or solvate thereof, wherein
$R^1$ is $C_1$-$C_4$ alkyl; and
$R^2$ is phenyl, or pyridinyl, wherein said phenyl is optionally substituted with one or more substituents, each independently selected from hydroxyl, alkoxy, halogen, haloalkoxy, and $-O(CH_2)_2OH$.

2. A compound as claimed in claim 1, wherein $R^1$ is methyl or butyl, and $R^2$ is phenyl or pyridinyl, wherein said phenyl is optionally substituted with one to three substituents, each independently selected from hydroxyl, methoxy, Cl, F, $-OCF_3$, and $-O(CH_2)_2OH$.

3. A compound as claimed in claim 1, wherein $R^1$ is butyl, and $R^2$ is phenyl substituted with three substituents, each independently selected from hydroxyl, methoxy, and Cl.

4. A compound according to claim 1 selected from the group consisting of:

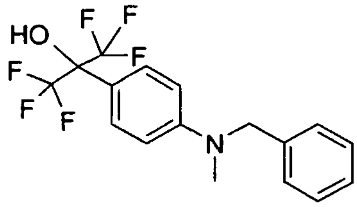 ;

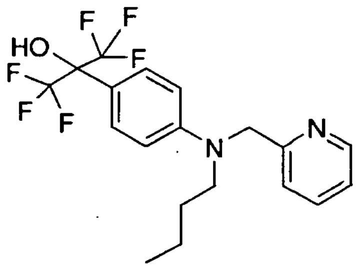 ;

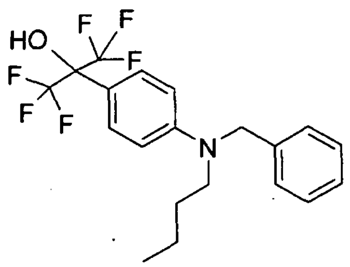
;

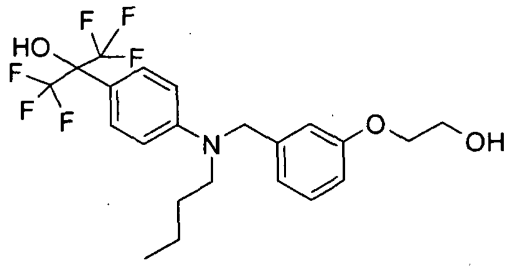
;

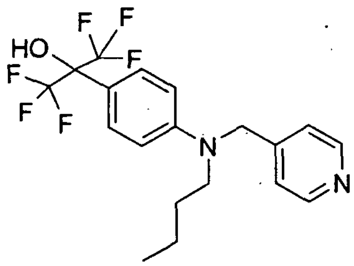
;

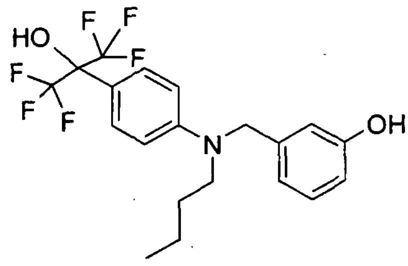
;

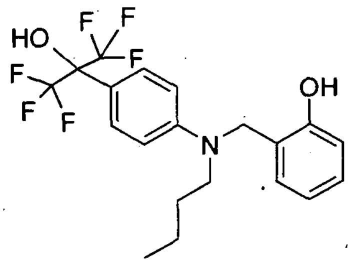
;

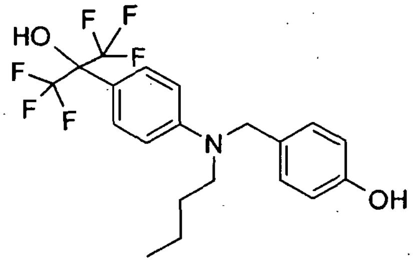
;

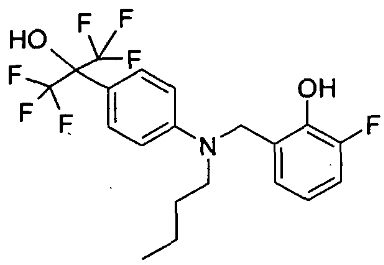
;

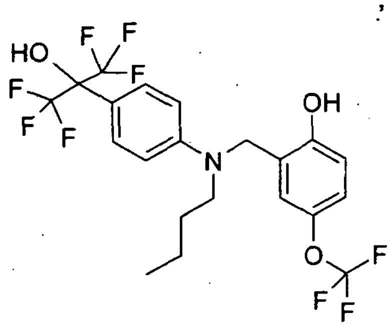
;

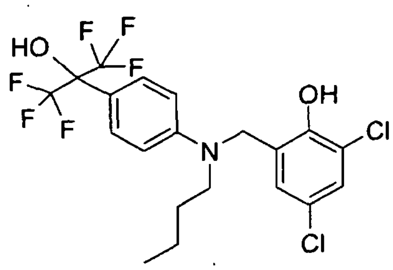
;

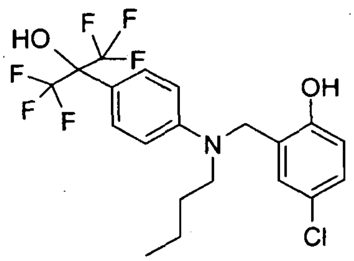
;

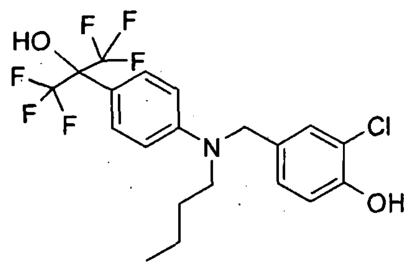
;

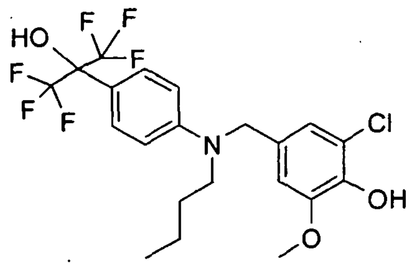
;

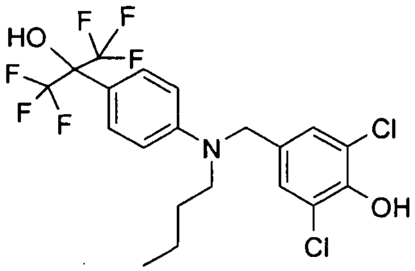
;
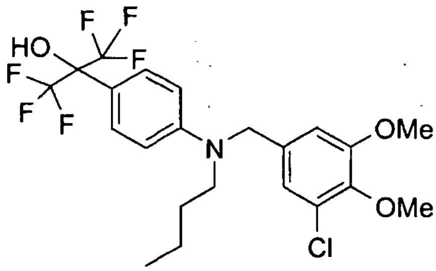
;
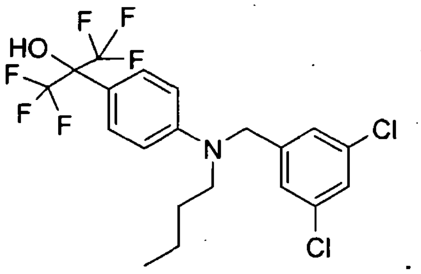
; and
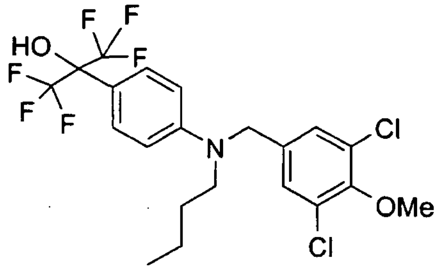
;

or a salt or solvate thereof.

**5.** A compound according to claim 1 of formula I-A:

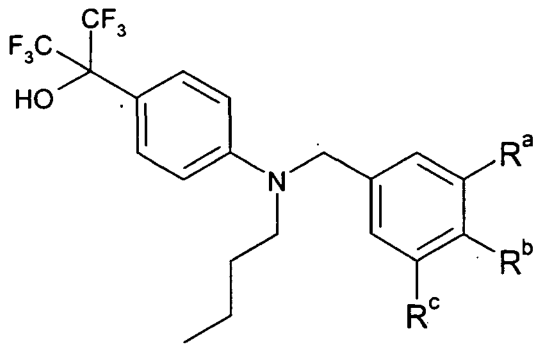
(I-A)

or a salt or solvate thereof, wherein
$R^a$ is Cl or methoxy;
$R^b$ is hydroxyl or methoxy; and
$R^c$ is Cl or methoxy.

**6.** A compound as claimed in claim 5, wherein $R^a$ is Cl, $R^b$ is hydroxyl, and $R^c$ is methoxy.

**7.** A compound as claimed in claim 5, wherein $R^a$ is Cl, $R^b$ is hydroxyl, and $R^c$ is Cl.

**8.** A compound as claimed in claim 5, wherein $R^a$ is methoxy, $R^b$ is methoxy, and $R^c$ is Cl.

**9.** A compound according to claim 5 selected from the group consisting of:

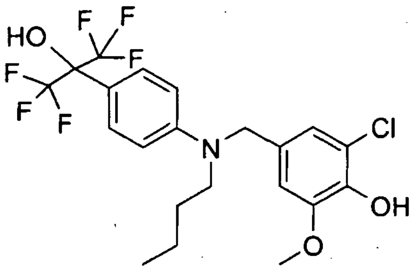 ; 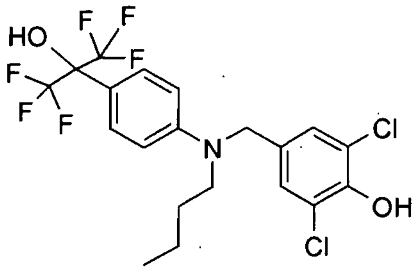 ;

and

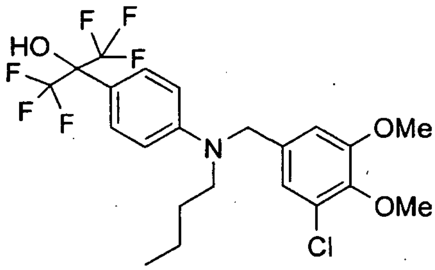 ;

or a salt or solvate thereof.

**10.** A pharmaceutical composition comprising a compound according to claims 1 to 9.

**11.** A compound according to claims 1 to 9 for use as an active therapeutic substance.

**12.** A compound according to claims 1 to 9 for use in the treatment of conditions or disorders that respond to LXR modulator activity.

**13.** A compound according to claims 1 to 9 for use in the treatment of diabetes mellitus, cardiovascular disease, dyslipidemia, artherosclerosis, peripheral vascular disease, inflammation, cancer, and diseases of the central nervous system. -

**14.** Use of a compound according to claims 1 to 9 in the manufacture of a medicament for use in the treatment of conditions or disorders that respond to LXR modulator activity.

**15.** Use of a compound according to any one of claims 1 to 9 in the manufacture of a medicament for use in the treatment of diabetes mellitus, cardiovascular disease, dyslipidemia, artherosclerosis, peripheral vascular disease, inflammation, cancer, and diseases of the central nervous system.

## Patentansprüche

**1.** Verbindung der Formel (I)

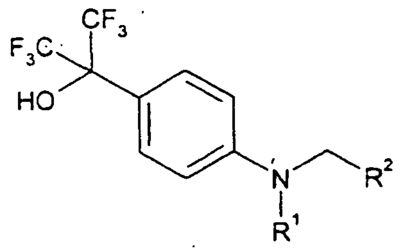

(I)

oder ein Salz oder Solvat davon, wobei
$R^1$ für $C_1$-$C_4$ Alkyl steht; und
$R^2$ Phenyl oder Pyridinyl ist, wobei das Phenyl gegebenenfalls mit einem oder mehreren jeweils unabhängig aus Hydroxyl, Alkoxy, Halogen, Halogenalkoxy und $-O(CH_2)_2OH$ ausgewählten Substituenten substituiert ist.

**2.** Verbindung gemäß Anspruch 1, wobei $R^1$ Methyl oder Butyl ist und $R^2$ Phenyl oder Pyridinyl ist, wobei das Phenyl gegebenenfalls mit einem bis drei jeweils unabhängig aus Hydroxyl, Methoxy, Cl, F, $-OCF_3$ und $-O(CH_2)_2OH$ ausgewählten Substituenten substituiert ist.

**3.** Verbindung gemäß Anspruch 1, wobei $R^1$ Butyl ist und $R^2$ mit drei jeweils unabhängig aus Hydroxyl, Methoxy und Cl ausgewählten Substituenten substituiertes Phenyl ist.

**4.** Verbindung gemäß Anspruch 1, ausgewählt aus der Gruppe:

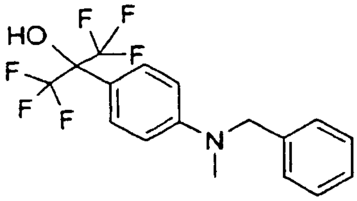

;

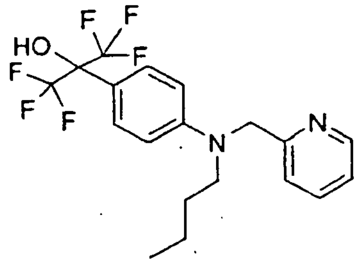

;

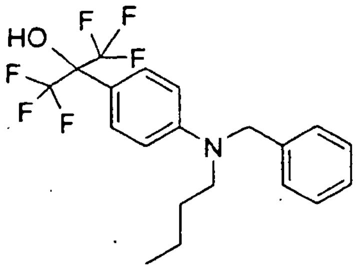

;

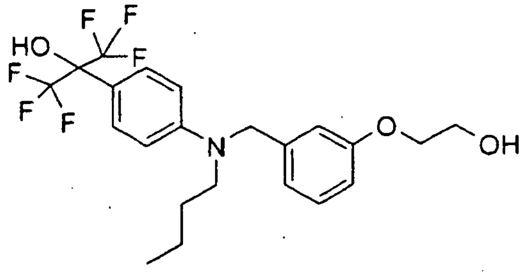

;

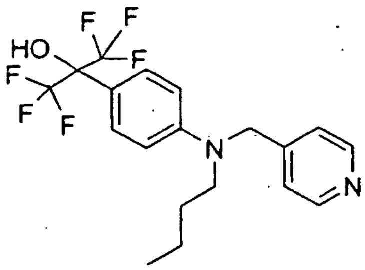

;

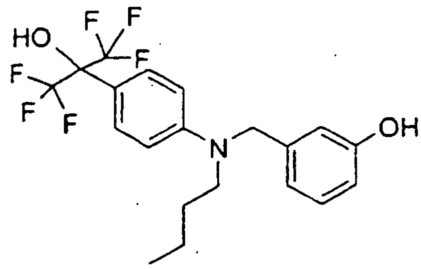

;

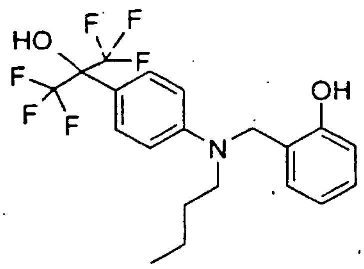

;

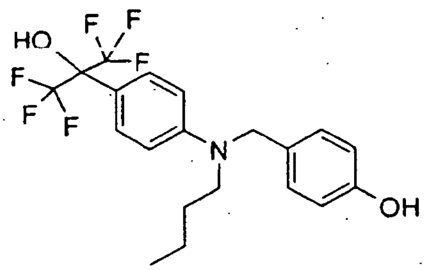

;

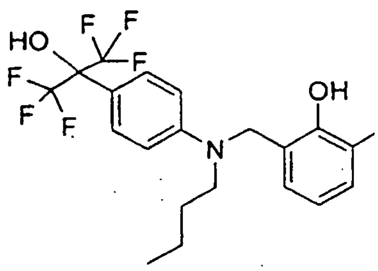

;

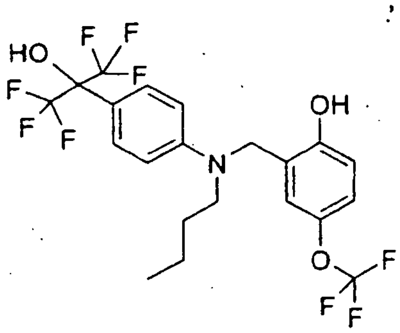

;

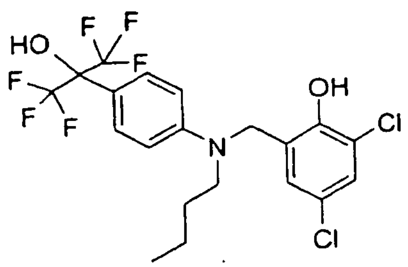

;

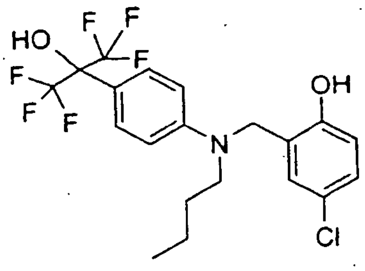

;

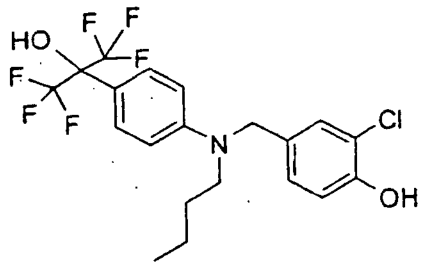

;

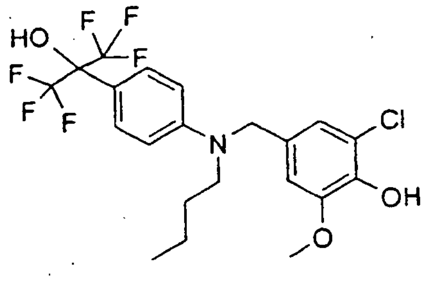

;

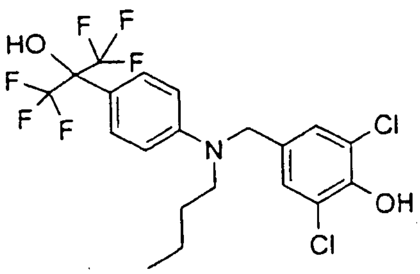

;

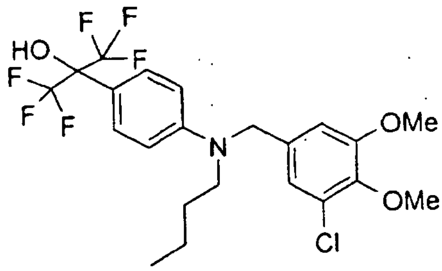

;

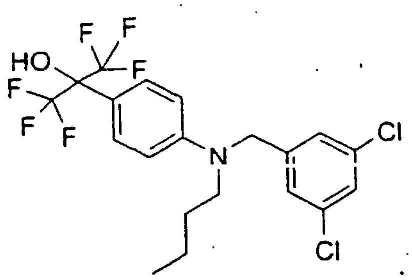

; und

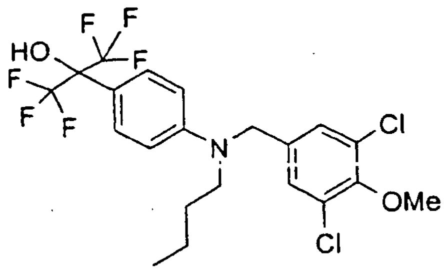

;

oder ein Salz oder Solvat davon.

**5.** Verbindung gemäß Anspruch 1 der Formel I-A:

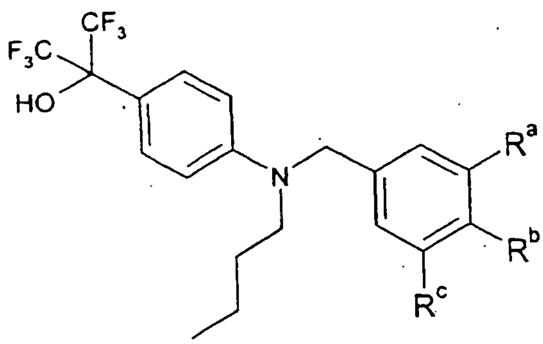

(I-A)

oder ein Salz oder Solvat davon, wobei
$R^a$ Cl oder Methoxy ist;
$R^b$ Hydroxyl oder Methoxy ist; und
$R^c$ Cl oder Methoxy ist.

**6.** Verbindung gemäß Anspruch 5, wobei $R^a$ für Cl steht, $R^b$ Hydroxyl ist und $R^c$ Methoxy ist.

**7.** Verbindung gemäß Anspruch 5, wobei $R^a$ für Cl steht, $R^b$ Hydroxyl ist und $R^c$ Cl ist.

**8.** Verbindung gemäß Anspruch 5, wobei $R^a$ Methoxy, $R^b$ Methoxy ist und $R^c$ Cl ist.

**9.** Verbindung gemäß Anspruch 5, ausgewählt aus der Gruppe:

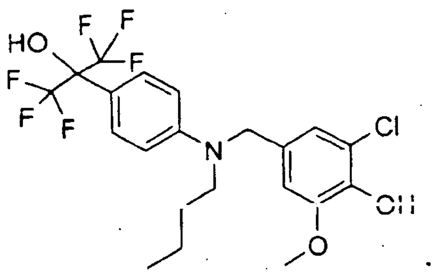
;
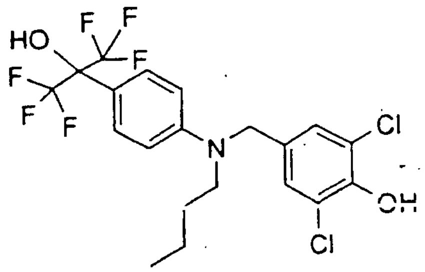
;

und

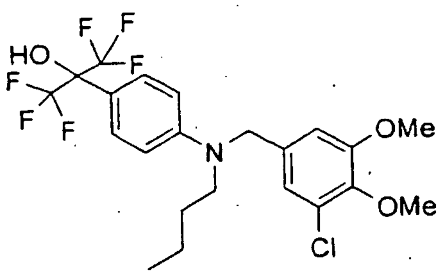
;

oder ein Salz oder Solvat davon.

**10.** Pharmazeutische Zusammensetzung, umfassend eine Verbindung gemäß den Ansprüchen 1 bis 9.

**11.** Verbindung gemäß den Ansprüchen 1 bis 9 zur Verwendung als wirksamer therapeutischer Stoff.

**12.** Verbindung gemäß den Ansprüchen 1 bis 9 zur Verwendung bei der Behandlung von Zuständen oder Störungen, die auf eine LXR Modulatoraktivität ansprechen.

**13.** Verbindung gemäß den Ansprüchen 1 bis 9 zur Verwendung bei der Behandlung von Diabetes Mellitus, kardiovaskulärer Erkrankung, Dyslipidämie, Arteriosklerose, peripherer arteriosklerotischer Gefäßerkrankung, Entzündung, Krebs und Erkrankungen des zentralen Nervensystems.

**14.** Verwendung einer Verbindung gemäß den Ansprüchen 1 bis 9 zur Herstellung eines Medikaments zur Verwendung bei der Behandlung von Zuständen oder Störungen, die auf eine LXR Modulatoraktivität ansprechen.

**15.** Verwendung einer Verbindung gemäß einem der Ansprüche 1 bis 9 zur Herstellung eines Medikaments zur Verwendung bei der Behandlung von Diabetes Mellitus, kardiovaskulärer Erkrankung, Dyslipidämie, Arteriosklerose, peripherer arteriosklerotischer Gefäßerkrankung, Entzündung, Krebs und Elkrankungen des zentralen Nervensystems.

**Revendications**

1. Composé de formule (I) :

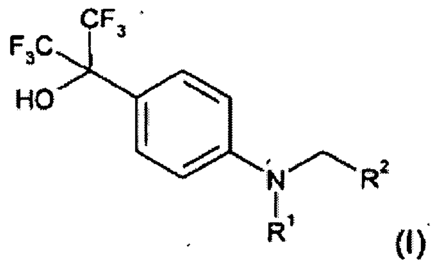

(I)

ou un de ses sels ou produits de solvatation, formule dans laquelle
$R^1$ représente un groupe alkyle en $C_1$ à $C_4$ ; et
$R^2$ représente un groupe phényle ou pyridinyle, ledit groupe phényle étant facultativement substitué avec un ou plusieurs substituants choisis chacun indépendamment entre des substituants hydroxyle, alkoxy, halogéno, halogénalkoxy et -O $(CH_2)_2OH$.

2. Composé suivant la revendication 1, dans lequel $R^1$ représente un groupe méthyle ou butyle et $R^2$ représente un groupe phényle ou pyridinyle, ledit groupe phényle étant facultativement substitué avec un à trois substituants choisis chacun indépendamment entre des substituants hydroxyle, méthoxy, Cl , F, $-OCF_3$ et $-O(CH_2)_2OH$.

3. Composé suivant la revendication 1, dans lequel $R^1$ représente un groupe butyle et $R^2$ représente un groupe phényle substitué avec trois substituants choisis chacun indépendamment entre des substituants hydroxyle, méthoxy et Cl.

4. Composé suivant la revendication 1, choisi dans le groupe consistant en :

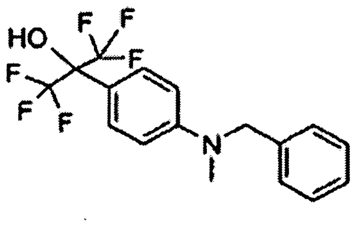
;
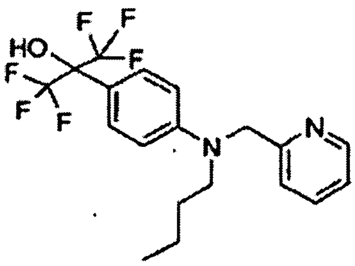
;

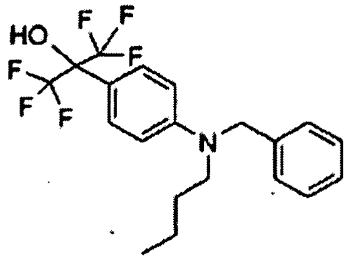
;

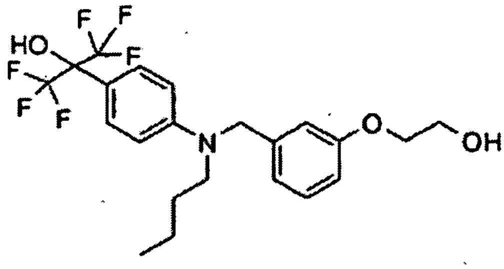
;

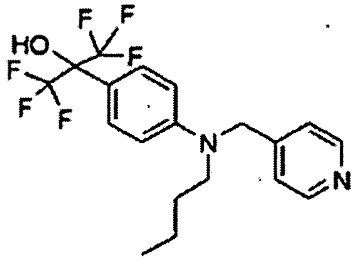
;

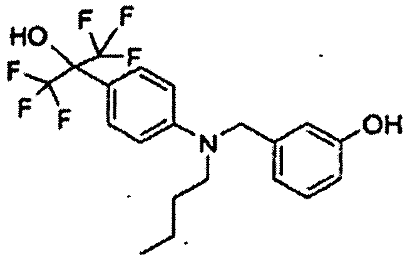
;

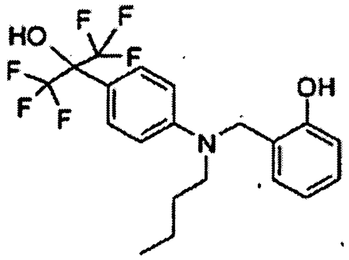
;

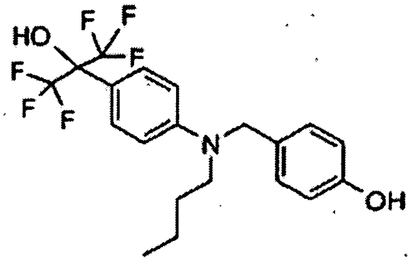
;

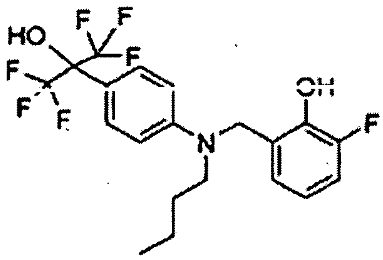
;

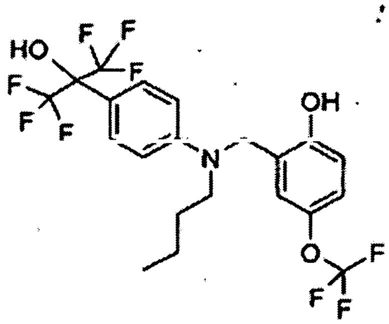
;

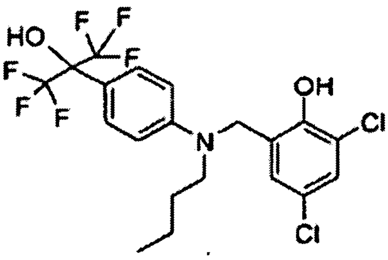
;

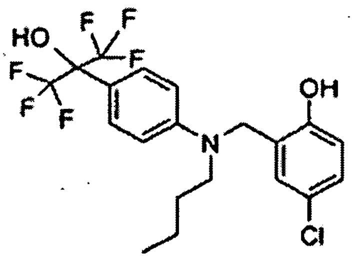
;

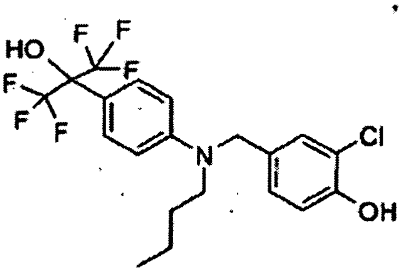
;

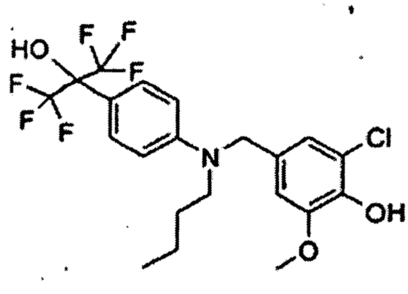
;

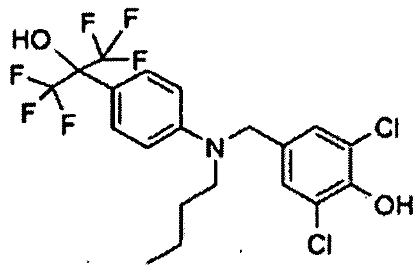

;

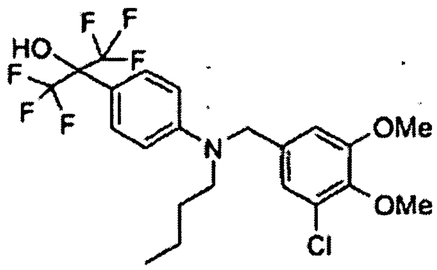

;

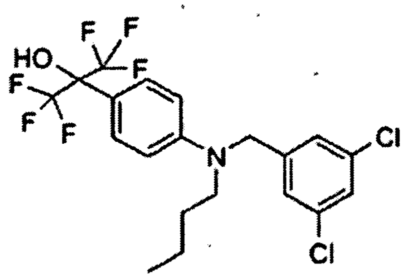

; et

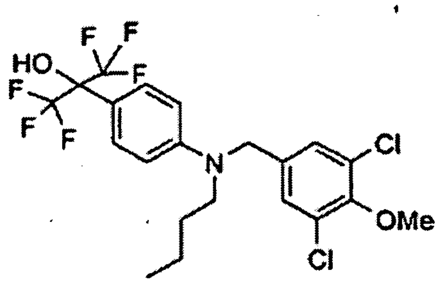

;

ou un de ses sels ou produits de solvatation.

**5.** Composé suivant la revendication 1, de formule I(A) :

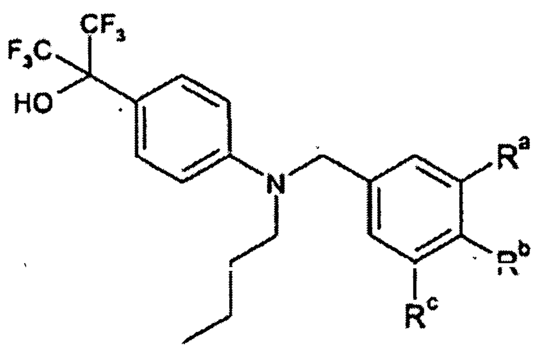

(I-A)

ou un de ses sels ou produits de solvatation, formule dans laquelle
$R^a$ représente un groupe Cl ou méthoxy ;
$R^b$ représente un groupe hydroxyle ou méthoxy ; et
$R^c$ représente un groupe Cl ou méthoxy.

**6.** Composé suivant la revendication 5, dans lequel $R^a$ représente un groupe Cl, $R^b$ représente un groupe hydroxyle et $R^c$ représente un groupe méthoxy.

**7.** Composé suivant la revendication 5, dans lequel $R^a$ représente un groupe Cl, $R^b$ représente un groupe hydroxyle et $R^c$ représente un groupe Cl.

**8.** Composé suivant la revendication 5, dans lequel $R^a$ représente un groupe méthoxy, $R^b$ représente un groupe méthoxy et $R^c$ représente un groupe Cl.

**9.** Composé suivant la revendication 5, choisi dans le groupe consistant en :

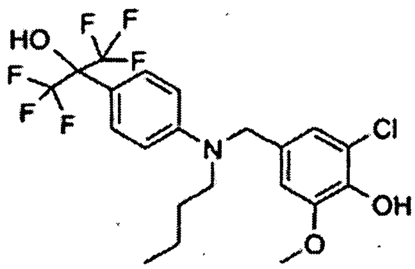 ; 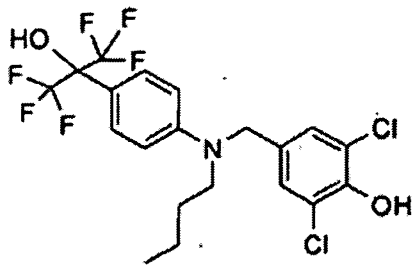 ;

et

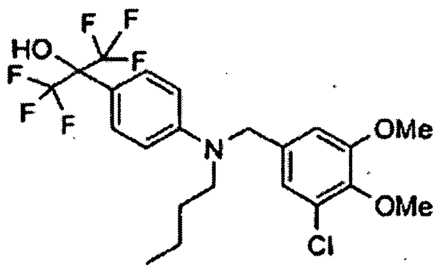 ;

ou de ses sels ou produits de solvatation.

**10.** Composition pharmaceutique comprenant un composé suivant les revendications 1 à 9.

**11.** Composé suivant les revendications 1 à 9, destiné à être utilisé comme substance thérapeutique active.

**12.** Composé suivant les revendications 1 à 9, destiné à être utilisé dans le traitement d'affections ou de troubles qui répondent à une activité de modulateur de LXR.

**13.** Composé suivant les revendications 1 à 9, destiné à être utilisé dans le traitement du diabète sucré, d'une maladie cardiovasculaire, de la dyslipidémie, de l'athérosclérose, d'une maladie vasculaire périphérique, de l'inflammation, du cancer et de maladies du système nerveux central.

**14.** Utilisation d'un composé suivant les revendications 1 à 9 dans la production d'un médicament destiné à être utilisé dans le traitement d'affections ou de troubles qui répondent à une activité de modulateur de LXR.

**15.** Utilisation d'un composé suivant l'une quelconque des revendications 1 à 9 dans la production d'un médicament destiné à être utilisé dans le traitement du diabète sucré, d'une maladie cardio-vasculaire, de la dyslipidémie, de l'athérosclérose, d'une maladie vasculaire périphérique, de l'inflammation, du cancer et de maladies du système nerveux central.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006032926 W
- US 60711267 B
- US 06032926 W


### Non-patent literature cited in the description


- **PEET, DJ et al.** *Curr. Opin. Genet. Dev.,* 1998, vol. 8 (5), 571-575
- **PEET, DJ et al.** *Cell,* 1998, vol. 93 (5), 693-704
- **SCHULTZ, JR et al.** *Gene Dev.,* 2000, vol. 14 (22), 2831-2838
- **COLLINS, JL et al.** *J. Med. Chem.,* 2002, vol. 45 (10), 1963-1966
- **TONTONOZ, P. et al.** *Mol. Endocrinol.,* 2003, vol. 17 (6), 985-993
- **JOSEPH, SB et al.** *Nat. Med.,* 2003, vol. 9 (2), 213-219
- **STULNIG, TM et al.** *Mol. Pharmacol.,* 2004, vol. 62 (6), 1299-1305
- **WANG, L et al.** *Proc. Natl. Acad. Sci. USA,* 2002, vol. 99 (21), 13878-13883
- Principles and Practice. Burger's Medicinal Chemistry And Drug Discovery. vol. 1
- *Pharmaceutical Research,* 1986, vol. 3 (6), 318
- **T. W. GREEN ; P. G. M. WUTS.** Protecting Groups in Organic Synthesis. John Wiley & Sons, 1991
- **E. L. ELIEL ; S. H. WILEN ; L. N. MANDER.** Stereochemistry of Organic Compounds. Wiley-Interscience, 1994

SEQUENCE LISTING

<110> SMITHKLINE BEECHAM CORPORATION
Collins, Jon Loren
Stewart, Eugene Lee
zuercher, William

Chao, Esther
wiethe, Robert
Caravella, Justin

<120> Chemical Compounds

<130> PR61599

<140> PCT/US2006/032926
<141> 2006-08-23

<150> US 60/711,267
<151> 2005-08-25

<160> 4

<170> PatentIn version 3.5
<210> 1
<211> 11
<212> PRT
<213> Artificial sequence

<220>
<223> Polyhistidine Tag

<400> 1

```
Met Lys Lys Gly His His His His His His Gly
1               5                   10
```

<210> 2
<211> 288
<212> PRT
<213> Homo sapiens

<400> 2

```
Met Lys Lys Gly His His His His His His Gly Pro Val Gly Pro Gln
1               5                   10                  15

Gly Ser Ser Ser Ser Ala Ser Gly Pro Gly Ala Ser Pro Gly Gly Ser
            20                  25                  30

Glu Ala Gly Ser Gln Gly Ser Gly Glu Gly Glu Gly Val Gln Leu Thr
        35                  40                  45

Ala Ala Gln Glu Leu Met Ile Gln Gln Leu Val Ala Ala Gln Leu Gln
    50                  55                  60

Cys Asn Lys Arg Ser Phe Ser Asp Gln Pro Lys Val Thr Pro Trp Pro
65                  70                  75                  80

Leu Gly Ala Asp Pro Gln Ser Arg Asp Ala Arg Gln Gln Arg Phe Ala
                85                  90                  95
```

His Phe Thr Glu Leu Ala Ile Ile Ser Val Gln Glu Ile Val Asp Phe
100 105 110

Ala Lys Gln Val Pro Gly Phe Leu Gln Leu Gly Arg Glu Asp Gln Ile
115 120 125

Ala Leu Leu Lys Ala Ser Thr Ile Glu Ile Met Leu Leu Glu Thr Ala
130 135 140

Arg Arg Tyr Asn His Glu Thr Glu Cys Ile Thr Phe Leu Lys Asp Phe
145 150 155 160

Thr Tyr Ser Lys Asp Asp Phe His Arg Ala Gly Leu Gln Val Glu Phe
165 170 175

Ile Asn Pro Ile Phe Glu Phe Ser Arg Ala Met Arg Arg Leu Gly Leu
180 185 190

Asp Asp Ala Glu Tyr Ala Leu Leu Ile Ala Ile Asn Ile Phe Ser Ala
195 200 205

Asp Arg Pro Asn Val Gln Glu Pro Gly Arg Val Glu Ala Leu Gln Gln
210 215 220

Pro Tyr Val Glu Ala Leu Leu Ser Tyr Thr Arg Ile Lys Arg Pro Gln
225 230 235 240

Asp Gln Leu Arg Phe Pro Arg Met Leu Met Lys Leu Val Ser Leu Arg
245 250 255

Thr Leu Ser Ser Val His Ser Glu Gln Val Phe Ala Leu Arg Leu Gln
260 265 270

Asp Lys Lys Leu Pro Pro Leu Leu Ser Glu Ile Trp Asp Val His Glu
275 280 285

<210> 3
<211> 14
<212> PRT
<213> Artificial Sequence

<220>
<223> Polyhistidine Tag

<400> 3

Met Arg Gly Ser His His His His His His Gly Met Ala Ser
1 5 10

<210> 4
<211> 276

<212> PRT
<213> Homo sapiens
<400> 4

Met Lys Lys Gly His His His His His His Gly Glu Glu Glu Gln Ala
1               5                   10                  15

His Ala Thr Ser Leu Pro Pro Arg Ala Ser Ser Pro Pro Gln Ile Leu
            20                  25                  30

Pro Gln Leu Ser Pro Glu Gln Leu Gly Met Ile Glu Lys Leu Val Ala
        35                  40                  45

Ala Gln Gln Gln Cys Asn Arg Arg Ser Phe Ser Asp Arg Leu Arg Val
    50                  55                  60

Thr Pro Trp Pro Met Ala Pro Asp Pro His Ser Arg Glu Ala Arg Gln
65                  70                  75                  80

Gln Arg Phe Ala His Phe Thr Glu Leu Ala Ile Val Ser Val Gln Glu
                85                  90                  95

Ile Val Asp Phe Ala Lys Gln Leu Pro Gly Phe Leu Gln Leu Ser Arg
            100                 105                 110

Glu Asp Gln Ile Ala Leu Leu Lys Thr Ser Ala Ile Glu Val Met Leu
        115                 120                 125

Leu Glu Thr Ser Arg Arg Tyr Asn Pro Gly Ser Glu Ser Ile Thr Phe
    130                 135                 140

Leu Lys Asp Phe Ser Tyr Asn Arg Glu Asp Phe Ala Lys Ala Gly Leu
145                 150                 155                 160

Gln Val Glu Phe Ile Asn Pro Ile Phe Glu Phe Ser Arg Ala Met Asn
                165                 170                 175

Glu Leu Gln Leu Asn Asp Ala Glu Phe Ala Leu Leu Ile Ala Ile Ser
            180                 185                 190

Ile Phe Ser Ala Asp Arg Pro Asn Val Gln Asp Gln Leu Gln Val Glu
        195                 200                 205

Arg Leu Gln His Thr Tyr Val Glu Ala Leu His Ala Tyr Val Ser Ile
    210                 215                 220

His His Pro His Asp Arg Leu Met Phe Pro Arg Met Leu Met Lys Leu
225                 230                 235                 240

Val Ser Leu Arg Thr Leu Ser Ser Val His Ser Glu Gln Val Phe Ala
                245                 250                 255

Leu Arg Leu Gln Asp Lys Lys Leu Pro Pro Leu Leu Ser Glu Ile Trp
            260                 265                 270

Asp Val His Glu
275

SEQUENCE LISTING

<110> SmithKline Beecham Corporation
COLLINS, Jon Loren
STEWART, Eugene Lee
ZUERCHER, william
CHAO, Esther
WIETHE, Robert
CARAVELLA, Justin

<120> Chemical Compounds

<130> PR61599

<140> PCT/US06/032926
<141>

<150> 60/711,267
<151> 2005-08-25

<160> 2

<170> FastSEQ for windows Version 4.0
<210> 1
<211> 288
<212> PRT
<213> unknown

<400> 1

Met Lys Lys Gly His His His His His His Gly Pro Val Gly Pro Gln
1 5 10 15
Gly Ser Ser Ser Ser Ala Ser Gly Pro Gly Ala Ser Pro Gly Gly Ser
20 25 30
Glu Ala Gly Ser Gln Gly Ser Gly Glu Gly Glu Gly Val Gln Leu Thr
35 40 45
Ala Ala Gln Glu Leu Met Ile Gln Gln Leu Val Ala Ala Gln Leu Gln
50 55 60
Cys Asn Lys Arg Ser Phe Ser Asp Gln Pro Lys Val Thr Pro Trp Pro
65 70 75 80
Leu Gly Ala Asp Pro Gln Ser Arg Asp Ala Arg Gln Gln Arg Phe Ala
85 90 95
His Phe Thr Glu Leu Ala Ile Ile Ser Val Gln Glu Ile Val Asp Phe
100 105 110
Ala Lys Gln Val Pro Gly Phe Leu Gln Leu Gly Arg Glu Asp Gln Ile
115 120 125
Ala Leu Leu Lys Ala Ser Thr Ile Glu Ile Met Leu Leu Glu Thr Ala
130 135 140
Arg Arg Tyr Asn His Glu Thr Glu Cys Ile Thr Phe Leu Lys Asp Phe
145 150 155 160
Thr Tyr Ser Lys Asp Asp Phe His Arg Ala Gly Leu Gln Val Glu Phe
165 170 175
Ile Asn Pro Ile Phe Glu Phe Ser Arg Ala Met Arg Arg Leu Gly Leu
180 185 190
Asp Asp Ala Glu Tyr Ala Leu Leu Ile Ala Ile Asn Ile Phe Ser Ala
195 200 205
Asp Arg Pro Asn Val Gln Glu Pro Gly Arg Val Glu Ala Leu Gln Gln
210 215 220
Pro Tyr Val Glu Ala Leu Leu Ser Tyr Thr Arg Ile Lys Arg Pro Gln
225 230 235 240
Asp Gln Leu Arg Phe Pro Arg Met Leu Met Lys Leu Val Ser Leu Arg
245 250 255
Thr Leu Ser Ser Val His Ser Glu Gln Val Phe Ala Leu Arg Leu Gln
260 265 270
Asp Lys Lys Leu Pro Pro Leu Leu Ser Glu Ile Trp Asp Val His Glu
275 280 285

<210> 2
<211> 276
<212> PRT
<213> unknown

<400> 2

```
Met Lys Lys Gly His His His His His His Gly Glu Glu Glu Gln Ala
1               5                   10                  15
His Ala Thr Ser Leu Pro Pro Arg Ala Ser Ser Pro Pro Gln Ile Leu
            20                  25                  30
Pro Gln Leu Ser Pro Glu Gln Leu Gly Met Ile Glu Lys Leu Val Ala
        35                  40                  45
Ala Gln Gln Gln Cys Asn Arg Arg Ser Phe Ser Asp Arg Leu Arg Val
    50                  55                  60
Thr Pro Trp Pro Met Ala Pro Asp Pro His Ser Arg Glu Ala Arg Gln
65                  70                  75                  80
Gln Arg Phe Ala His Phe Thr Glu Leu Ala Ile Val Ser Val Gln Glu
                85                  90                  95
Ile Val Asp Phe Ala Lys Gln Leu Pro Gly Phe Leu Gln Leu Ser Arg
            100                 105                 110
Glu Asp Gln Ile Ala Leu Leu Lys Thr Ser Ala Ile Glu Val Met Leu
        115                 120                 125
Leu Glu Thr Ser Arg Arg Tyr Asn Pro Gly Ser Glu Ser Ile Thr Phe
    130                 135                 140
Leu Lys Asp Phe Ser Tyr Asn Arg Glu Asp Phe Ala Lys Ala Gly Leu
145                 150                 155                 160
Gln Val Glu Phe Ile Asn Pro Ile Phe Glu Phe Ser Arg Ala Met Asn
                165                 170                 175
Glu Leu Gln Leu Asn Asp Ala Glu Phe Ala Leu Leu Ile Ala Ile Ser
            180                 185                 190
Ile Phe Ser Ala Asp Arg Pro Asn Val Gln Asp Gln Leu Gln Val Glu
        195                 200                 205
Arg Leu Gln His Thr Tyr Val Glu Ala Leu His Ala Tyr Val Ser Ile
    210                 215                 220
His His Pro His Asp Arg Leu Met Phe Pro Arg Met Leu Met Lys Leu
225                 230                 235                 240
Val Ser Leu Arg Thr Leu Ser Ser Val His Ser Glu Gln Val Phe Ala
                245                 250                 255
Leu Arg Leu Gln Asp Lys Lys Leu Pro Pro Leu Leu Ser Glu Ile Trp
            260                 265                 270
Asp Val His Glu
        275
```